(19) Europäisches Patentamt / European Patent Office / Office européen des brevets

(11) **EP 3 246 043 A1**

(12) **EUROPEAN PATENT APPLICATION**
published in accordance with Art. 153(4) EPC

(43) Date of publication:
**22.11.2017 Bulletin 2017/47**

(21) Application number: **15857606.6**

(22) Date of filing: **03.11.2015**

(51) Int Cl.:
*A61K 38/17* (2006.01)          *A61P 1/16* (2006.01)
*A61P 3/04* (2006.01)          *A61P 5/50* (2006.01)

(86) International application number:
**PCT/CN2015/093726**

(87) International publication number:
**WO 2016/070798 (12.05.2016 Gazette 2016/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority:  03.11.2014  CN 201410610777
                15.01.2015  CN 201510021469

(71) Applicants:
• **Tsinghua University**
  **Haidian District**
  **Beijing 100084 (CN)**
• **Beijing Protgen Ltd.**
  **Beijing 100085 (CN)**

(72) Inventors:
• **LUO, Yongzhang**
  **Beijing 100084 (CN)**
• **WANG, Hui**
  **Beijing 100084 (CN)**
• **LI, Hui**
  **Beijing 100085 (CN)**
• **LU, Xinan**
  **Beijing 100084 (CN)**
• **FU, Yan**
  **Beijing 100084 (CN)**
• **ZHAN, Shunli**
  **Beijing 100085 (CN)**
• **ZHOU, Daifu**
  **Beijing 100085 (CN)**

(74) Representative: **Ström & Gulliksson AB**
  **P.O. Box 4188**
  **203 13 Malmö (SE)**

(54) **DRUG FOR INHIBITING ADIPOSE CELL DIFFERENTIATION AND INSULIN RESISTANCE**

(57)     The present invention provides use of endostatin or a functional variant thereof in the preparation of a medicament for treating dietary obesity, non-alcoholic fatty liver disease, insulin resistance or glucose intolerance. In the embodiments of the present invention, the functional variant may be YH-16, mES, mYH-16, m003, m007, mZ101, or the like.

EP 3 246 043 A1

**Description**

**Field of the Invention**

The present invention relates to a novel function of endostatin. Specifically, the present invention discloses that endostatin significantly inhibits adipocyte differentiation and alleviates insulin resistance. The present invention also provides a new use of endostatin in treating dietary obesity, non-alcoholic fatty liver disease, insulin resistance, glucose intolerance, and other diseases.

**Background of the Invention**

The accumulation of fat could cause expansion of adipose tissue, which means increased number and volume of adipocytes along with angiogenesis (Cristancho AG et al., Nat Rev Mol Cell Biol 2011; 12:722-734; Daquinag AC et al., Trends Pharmacol Sci 2011; 32:300-307).

In 1997, Folkman's Laboratory discovered an endogenous vascular inhibitor endostatin (ES), which could be directly targeted to vascular endothelial cells, with angiogenesis inhibition and tumor treatment activities (O'Reilly MS et al., Cell 1997; 88:277-285; Boehm T. et al., Nature 197; 390:404-407).

YH-16 is an ES variant obtained by adding nine additional amino acids (MGGSHHHHH) at N-terminal of ES, which acquired national first-in-class new drug certificate in 2005 for the treatment of non-small cell lung cancer (Fu Y et al., IUBMB Life 2009; 61:613-626; Wang J et al., Zhongguo fei ai za zhi 2005; 8:283-290; Han B et al., J Thorac Oncol 2011; 6(6):1104-1109). PEG-modified ES and YH-16 were named as mES and mYH-16 respectively and were obtained by the modification of ES or YH-16 molecule with a 20kDa monomethoxy polyethylene glycol propionaldehyde (mPEG-ALD). The coupling sites were activated aldehyde group of mPEG-ALD and N-terminal $\alpha$-amino group of ES or YH-16.

It was reported that angiogenic inhibitor can inhibit obesity through inhibiting angiogenesis in adipose tissue (Rupnick MA et al., Proc Natl Acad Sci U S A 2002; 99:10730-10735; Kim MY et al., Int J Obes (Lond). 2010; 34:820-830). In 2002, Folkman's Laboratory reported several different vascular inhibitors that can inhibit hereditary obesity in mice, including ES (Rupnick MA et al., Proc Natl Acad Sci U S A 2002; 99:10730-10735).

The increase in number of adipocytes directly depends on adipocyte differentiation (Cristancho AG et al., Nat Rev Mol Cell Biol 2011; 12:722-734), which is a very complicated regulatory process. Studies have shown that peroxisome proliferator-activated receptor gamma (PPAR$\gamma$) is the central regulatory factor in regulating adipocyte differentiation (Tang QQ et al., Annu Rev Biochem 2012; 81:715-736), which can regulate adipocyte differentiation by regulating the expression of downstream adipocyte phenotype control genes (including CD36, ap2, Glut4, LPL and LXR, etc.) (Cristancho AG et al., Nat Rev Mol Cell Biol 2011; 12:722-734; Lee J et al., J Cell Biochem 2012; 113:2488-2499).

A lot of epidemiological studies have shown that obesity can cause metabolic disorders, and is an important clinical manifestation of metabolic syndrome, but also an important risk factor in causing non-alcoholic fatty liver disease, insulin resistance, glucose intolerance, and type II diabetes (Malik VS et al., Nat Rev Endocrinol 2013; 9:13-27).

**Summary of the Invention**

The present invention relates to a novel function of the known vascular inhibitor protein endostatin (ES), namely the activity in inhibiting adipocyte differentiation, and provides, based on this novel function, a novel use of ES in the treatment of metabolic disorders such as dietary obesity, non-alcoholic fatty liver disease, insulin resistance, and glucose intolerance, etc.

The inventor discovered that ES can inhibit adipocyte differentiation by acting directly on preadipocytes and inhibiting the expression of central regulatory factors PPAR$\gamma$1 and/or PPAR$\gamma$2 in adipocyte differentiation.

The inventor discovered that ES can inhibit weight gain induced by high-fat diet in mice by inhibiting the accumulation of fat in mice.

The inventor discovered that ES can inhibit the increase in liver weight and fat deposition induced by high-fat diet in mice, thereby preventing and treating hepatic adipose infiltration.

The inventor also discovered that ES can enhance the response of mice to insulin by increasing the phosphorylation of Akt, so as to improve insulin resistance and glucose intolerance in mice.

The inventor also discovered that the variants of ES, such as YH-16, 003, 007 and Z101, have activity comparable to that of ES in above experiments. Polyethylene glycol (PEG)-modified ES and its variants YH-16, 003, 007 and Z101 (mES, mYH-16, m003, m007 and mZ101) have similar activity to the unmodified protein.

**Brief Description of the Drawings**

Figure 1A shows that ES and its variant YH-16 significantly inhibited weight gain induced by high-fat diet in mice. *** means P< 0.001.

Figure 1B shows that ES and its variant YH-16 significantly inhibited the increase in adipose tissue weight induced by high-fat diet in mice. * means P< 0.05, *** means P< 0.001.

Figure 1C shows that ES and its variant YH-16 had no effect on the weight of lungs, heart and kidneys in mice with high-fat diet.

Figure 2A shows that ES and its variant YH-16 significantly inhibited the increase in liver weight induced by high-fat diet in mice. * means P< 0.05, ** means P< 0.01.

Figure 2B shows that ES and its variant YH-16 treated group mice liver tissue slices.

Figure 2C shows that ES and its variant YH-16 significantly inhibited liver fat deposition induced by high-fat diet in mice. *** means P< 0.001.

Figure 3A shows that ES and its variant YH-16 significantly improved insulin resistance in mice. *** means P< 0.001.

Figure 3B shows that ES and its variant YH-16 significantly improved glucose tolerance in mice. *** means P< 0.001.

Figure 3C shows that ES significantly increased the phosphorylation level of Akt, the downstream factor of insulin signaling pathway.

Figure 4A shows that ES and its variant YH-16, PEG-modified ES and its variant YH-16 (mES and mYH-16) directly inhibited adipocyte differentiation.

Figure 4B shows that quantitative statistical results of inhibition of adipocyte differentiation by ES and its variant YH-16, PEG-modified ES and its variant YH-16 (mES and mYH-16). *** means P< 0.001

Figure 4C shows that ES and its variant YH-16, PEG-modified ES and its variant YH-16 (mES and mYH-16) significantly inhibited protein expression of adipocyte differentiation central regulatory factors PPARγ1/2.

Figure 4D shows that ES inhibited mRNA expression level of adipocyte differentiation central regulatory factors PPARγ1/2. * means P< 0.05, *** means P< 0.001.

Figure 5A shows that PEG-modified ES and its variants 003, 007 (mES, m003, and m007) significantly inhibited weight gain induced by high-fat diet in mice. *** means P< 0.001.

Figure 5B shows that PEG-modified ES and its variants 003, 007 (mES, m003, and m007) significantly inhibited the increase in adipose tissue weight induced by high-fat diet in mice. * means P< 0.05, *** means P< 0.001.

Figure 5C shows that PEG-modified ES and its variants 003, 007 (mES, m003, and m007) had no effect on the weight of lungs, heart and kidneys in mice with high-fat diet.

Figure 6A shows that PEG-modified ES and its variants 003, 007 (mES, m003, and m007) significantly inhibited the increase liver weight induced by high-fat diet in mice. * means P< 0.05, ** means P< 0.01.

Figure 6B shows that PEG-modified ES and its variants 003, 007 (mES, m003, and m007) treated group mice liver tissue slices.

Figure 6C shows that PEG-modified ES and its variants 003, 007 (mES, m003, and m007) significantly inhibited liver fat deposition induced by high-fat diet in mice. *** means P< 0.001.

Figure 7A shows that PEG-modified ES and its variants 003, 007 (mES, m003, and m007) directly inhibited adipocyte differentiation.

Figure 7B shows that quantitative results of inhibition of adipocyte differentiation by PEG-modified ES and its variants 003, 007 (mES, m003, and m007). *** means P< 0.001.

Figure 8A shows that PEG-modified ES variant Z101 (mZ101) significantly inhibited weight gain induced by high-fat diet in mice. ** means P< 0.01, *** means P< 0.001.

Figure 8B shows that PEG-modified ES variant Z101 (mZ101) significantly inhibited the increase in adipose tissue weight induced by high-fat diet in mice. *** means P< 0.001.

Figure 8C shows that PEG-modified ES variant Z101 (mZ101) significantly inhibited the increase in liver weight induced by high-fat diet in mice. * means P< 0.05.

Figure 8D shows that PEG-modified ES variant Z101 (mZ101) had no effect on the weight of lungs, heart and kidneys in mice with high-fat diet.

Figure 9A shows that PEG-modified ES variant Z101 (mZ101) directly inhibited adipocyte differentiation.

Figure 9B shows that quantitative results of inhibition of adipocyte differentiation by PEG-modified ES variant Z101 (mZ101). *** means P< 0.001.

Figure 10A shows that PEG-modified ES variants 009 and S03 (m009 and mS03) significantly inhibited weight gain induced by high-fat diet in mice. ** means P< 0.01, *** means P< 0.001.

Figure 10B shows that PEG-modified ES variants 009 and S03 (m009 and mS03) significantly inhibited the increase in adipose tissue weight induced by high-fat diet in mice. *** means P< 0.001.

Figure 10C shows that PEG-modified ES variants 009 and S03 (m009 and mS03) significantly inhibited the increase in liver weight induced by high-fat diet in mice. * means P< 0.05.

Figure 10D shows that PEG-modified ES variants 009 and S03 (m009 and mS03) had no effect on the weight of lungs, heart and kidneys in mice with high-fat diet.

Figure 11A shows that PEG-modified ES variants 009 and S03 (m009 and mS03) directly inhibited adipocyte differentiation.

Figure 11B shows that quantitative results of inhibition of adipocyte differentiation by PEG-modified ES variants 009 and S03 (m009 and mS03). *** means P< 0.001.

Figure 12A shows that PEG-modified ES variants 36 and 249 (m36 and m249) significantly inhibited weight gain induced by high-fat diet in mice. ** means P< 0.01, *** means P< 0.001.

Figure 12B shows that PEG-modified ES variants 36 and 249 (m36 and m249) significantly inhibited the increase in adipose tissue weight induced by high-fat diet in mice. * means P< 0.05, ** means P< 0.01, *** means P< 0.001.

Figure 12C shows that PEG-modified ES variants 36 and 249 (m36 and m249) significantly inhibited the increase in liver weight induced by high-fat diet in mice. * means P< 0.05, ** means P< 0.01.

Figure 12D shows that PEG-modified ES variants 36 and 249 (m36 and m249) had no effect on the weight of lungs, heart and kidneys in mice with high-fat diet.

Figure 13 shows the amino acid sequence of ES variant 003.

Figure 14 shows the amino acid sequence of ES variant 007.

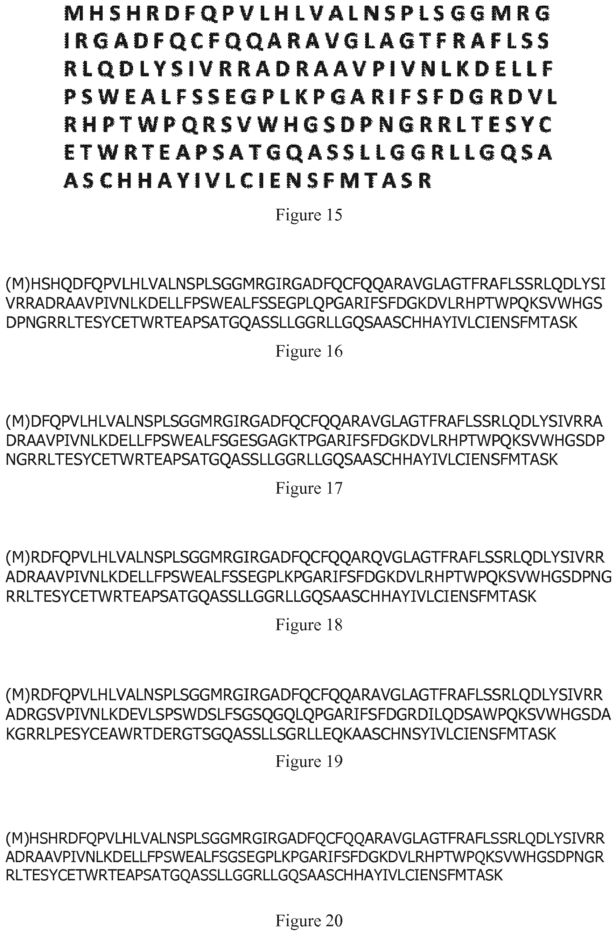

Figure 15 shows the amino acid sequence of ES variant Z101.

Figure 16 shows the amino acid sequence of ES variant 009.

Figure 17 shows the amino acid sequence of ES variant S03.

Figure 18 shows the amino acid sequence of ES variant 36.

Figure 19 shows the amino acid sequence of ES variant 249.

Figure 20 shows the amino acid sequence of ES.

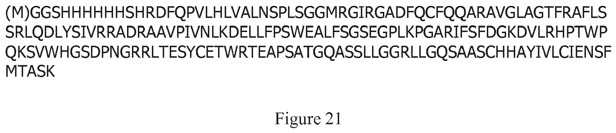

Figure 21 shows the amino acid sequence of ES variant YH-16.

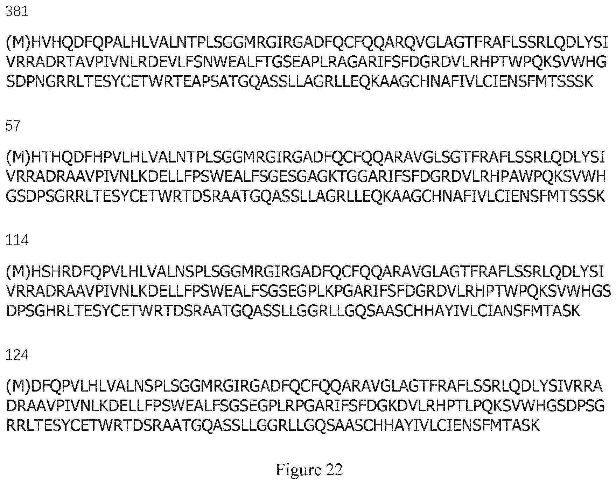

Figure 22 shows the amino acid sequences of ES variants 381, 57, 114, and 124.

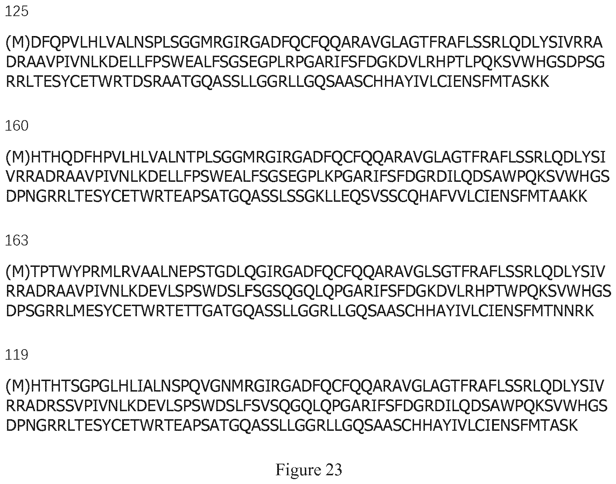

Figure 23 shows the amino acid sequences of ES variants 125, 160, 163, and 119.

**Detailed Description of the Invention**

The present invention provides use of endostatin or a functional variant thereof in preparing a medicament for treating dietary obesity, non-alcoholic fatty liver disease, insulin resistance or glucose intolerance.

The present invention provides use of endostatin or a functional variant thereof in preparing a medicament for preventing adipocyte differentiation.

In some embodiments, the said functional variant may be YH-16, 003, 007, Z101, ES006, ES008, ES011, S02, S09, Z006, Z008, ZN1, 009, S03, 36, 249, 381, 57, 114, 124, 125, 160, 163, 119, mES, mYH-16, m003, m007, mZ101, mES006, mES008, mES011, mS02, mS09, mZ006, mZ008, mZN1, m009, mS03, m36, m249, m381, m57, m114, m124, m125, m160, m163, or m119. In preferred embodiments of the present invention, the said functional variant may be YH-16, 003, 007, Z101, 009, S03, 36, 249, mES, mYH-16, m003, m007, mZ101, m009, mS03, m36, or m249.

As used herein, the terms "functional variant" and "functional variants" include endostatin mutants having substitution, deletion or addition of one or more (for example 1 to 5, 1 to 10 or 1 to 15, specifically, for example, 1, 2, 3, 4, 5, 6, 7, 8, 9, 10,12 or even more) amino acids in the amino acid sequence, and derivatives obtained by chemically modifying endostatin or its mutants, for example, PEG modification. The mutants and derivatives have substantially the same activity of inhibiting adipocyte differentiation as endostatin. For example, PEG-modified ES and YH-16 are named as mES and mYH-16 respectively, and are obtained by the modification of ES or YH-16 with a 20kDa monomethoxy polyethylene glycol propionaldehyde (mPEG-ALD). The coupling sites are activated aldehyde group of mPEG-ALD and N-terminal $\alpha$-amino group of ES or YH-16 (other ES mutants and PEG-modified derivatives of the mutants are similarly modified and named). For example, in the embodiments of the present invention, YH-16, 003, 007, Z101, 009, S03, 36 and 249 are the particularly preferred mutants of endostatin; mES, mYH-16, m003, m007, mZ101, m009, mS03, m36 and m249 are preferred derivatives of ES, YH-16, 003, 007, Z101, 009, S03, 36 and 249 respectively. PCT application PCT/CN2012/081210 (which is hereby incorporated by reference in its entirety) provides various mutants of endostatin such as ES006, ES008, ES011, S02, S09, Z006, Z008, and ZN1 etc. The terms "functional variant", "functional variants", "variant", or "variants" in this context cover the mutants and derivatives of endostatin.

The present invention also provides a method for treating dietary obesity, non-alcoholic fatty liver disease, insulin resistance or glucose intolerance, comprising administering to a subject a therapeutically effective amount of endostatin or a functional variant thereof.

As used herein, the term "therapeutically effective amount" refers to an amount of active compound sufficient to cause a biological or medical response desired by the clinician in a subject. The "therapeutically effective amount" of endostatin or a functional variant thereof can be determined by those skilled in the art depending on factors such as route of administration, weight, age and condition of the subject, and the like. For example, a typical daily dose may range from 0.01mg to 100mg of active ingredient per kg of body weight.

The medicament provided in the present invention can be prepared into a clinically acceptable dosage form such as a powder, an injection and the like, and can be administered by conventional means such as injection.

The present invention also provides a method for inhibiting adipocyte differentiation; comprising administering to a subject a therapeutically effective amount of endostatin or a functional variant thereof.

The present invention also provides a medicament for the treatment of dietary obesity, non-alcoholic fatty liver disease, insulin resistance or glucose intolerance, including endostatin or a functional variant thereof as active ingredient.

**Dietary obesity** refers to obesity caused by excess calories stored in the form of fat in the body when the calories in the diet exceed the body's energy consumption.

**Non-alcoholic fatty liver disease** (NAFLD) is metabolic stress induced liver injury closely correlated with insulin resistance and genetic susceptibility. Its pathological phenotype is similar to that of alcoholic liver disease (ALD), but patients have no history of excessive drinking.

**Insulin resistance,** also known as insulin tolerance, which means the insusceptibility of body to insulin so that the promoting effect of insulin on the intake and utilization of glucose is below normal level. In other words, the body requires higher concentration of insulin to respond to insulin. Insulin resistance induced high level of insulin and high glucose in the plasma usually lead to metabolic syndrome, gout and type II diabetes.

**Glucose intolerance** is the decline in the capability to adjust blood glucose level due to the reduced glucose metabolism of the body, manifested in that the blood glucose level cannot be timely adjusted back to normal after large intake of glucose. It can develop into diabetes if not interfered timely.

**Inhibitory ratio of mice body weight** = (1 - increase of body weight in the drug treated group/increase of body weight in the group with high-fat diet) ×100%.

**Inhibitory ratio of mice fat storage** = (1 - adipose tissue weight in the drug treated group/adipose tissue weight in the group with high-fat diet) ×100%.

**Inhibitory ratio of mouse liver weight** = (1 - liver weight in the drug treated group/liver weight in the group with high-fat diet) ×100%.

**Inhibitory ratio of mouse liver fat deposition** = (1 - hepatic cytoplasmic vacuolar ratio in the drug treated group/hepatic cytoplasmic vacuolar ratio in the group with high-fat diet) ×100%.

The ES and variants thereof utilized in the examples of the present invention were all provided by Beijing Protgen Ltd.

**Examples**

**Example 1 ES and YH-16 significantly inhibited weight gain induced by high-fat diet in mice**

A total of 24 healthy C57BL/6 mice (7-week old, male, purchased from Beijing Vital River Laboratory Animal Technology Company) were divided into 4 groups with 8 mice in each group and treated as follows:

    Group 1: normal diet group;
    Group 2: high-fat diet group;
    Group 3: high-fat diet + ES treated group (drug treated group);
    Group 4: high-fat diet + YH-16 treated group (drug treated group).

Mice in normal diet group were fed with feedstuff in which 10% calories come from fat component (D12450J, Research Diets, USA); mice in high-fat diet group were fed with feedstuff in which 60% calories come from fat component (D12492J, Research Diets, USA).

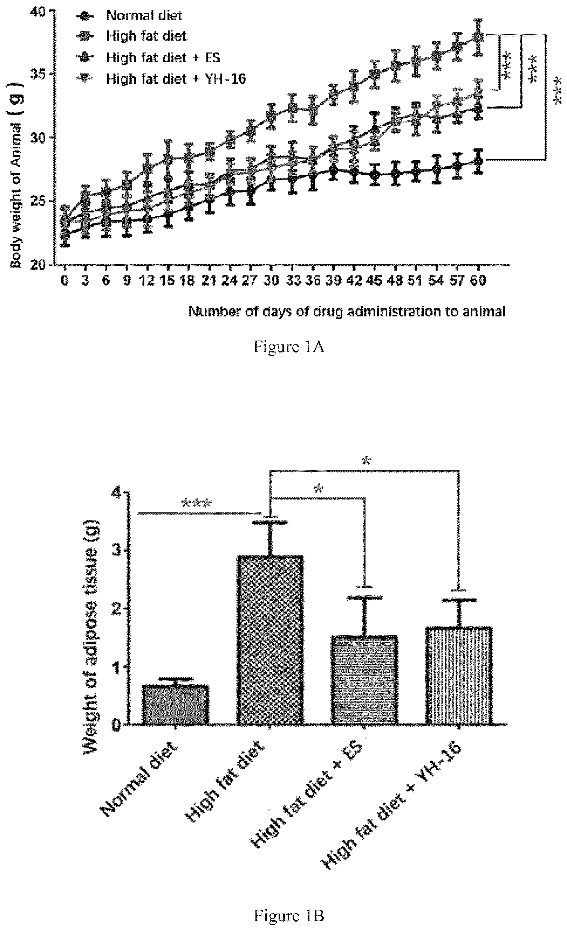

Route of administration: in an injection period of 60 days, group 3 and group 4 were injected intraperitoneally once a day with ES or YH-16 (Protgen) at a dose of 12 mg/kg/day, group 2 was injected intraperitoneally with equal volume of saline, group 1 was not injected. The first day of injection was set as day 0, and the last administration was carried out on day 59. The mice were weighed once every three days, and the last measurement was performed on day 60 (i.e., the day after the last administration), and the body weight curves were plotted (Fig. 1A). The results showed that both ES and YH-16 significantly inhibited weight gain due to high-fat diet, and the inhibition ratios were 37.5%, and 30.6% respectively (Table 1).

After completion of the glucose tolerance test on day 61, the mice were sacrificed and whole body adipose tissues were isolated and weighed (Fig. 1B, Table 1). The results showed that the adipose tissue weight of mice in group with ES or YH-16 treatment was remarkably lower than that in high-fat diet group without drug treatment. The inhibitory ratios of ES and YH-16 on fat accumulation induced by high-fat diet in mice were 47.7%, and 42.2%, respectively (Table 1).

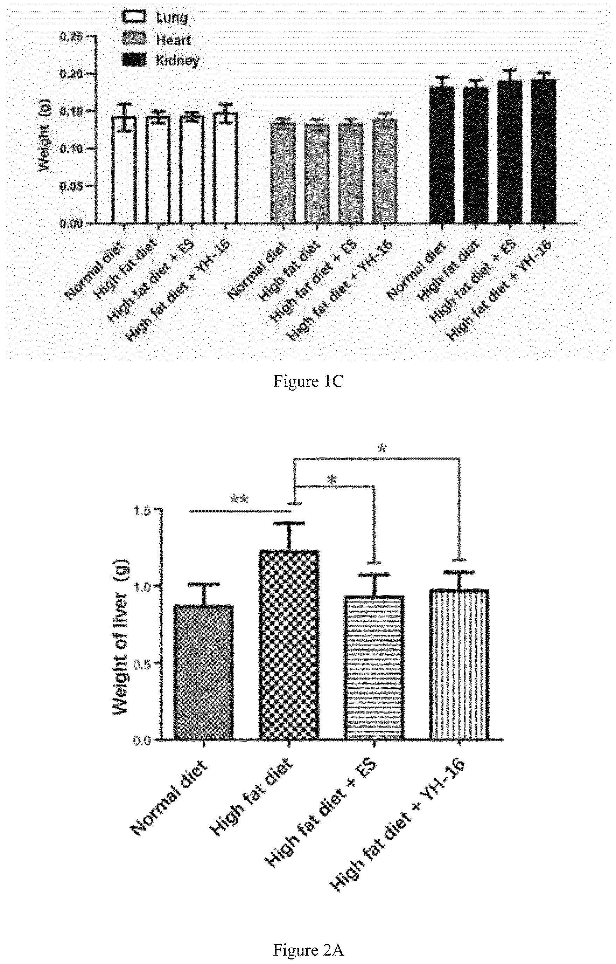

Lungs, heart and kidneys were isolated from mice and weighed (Fig. 1C, Table 1). The results showed that there was no significant difference in the weight of lungs, heart and kidneys among the mice in all four groups, indicating that ES and YH-16 had no effect on lungs, heart and kidneys of mice.

**Example 2 ES and YH-16 significantly inhibited the increase in liver weight and fat deposition induced by high-fat diet in mice**

From the mice in Example 1, after completion of the glucose tolerance test on day 61, the liver tissues were removed and weighed (Fig. 2A, Table 1). ES and YH-16 inhibited the increase in liver weight induced by high-fat diet in mice, with inhibition ratios of 23.8% and 20.5%, respectively.

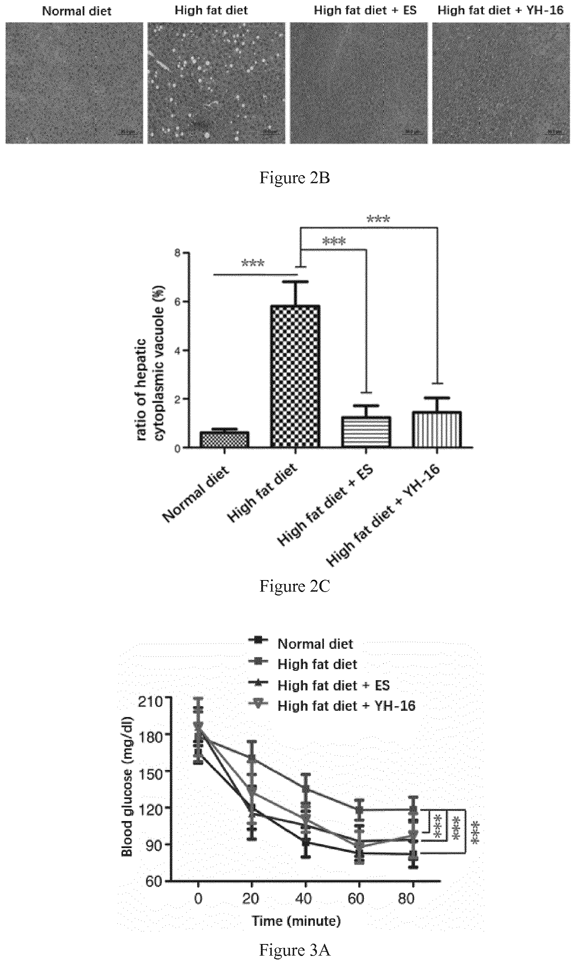

The liver tissues were fixed and embedded in paraffin, then sliced into 8 μm thick sections. Then the liver tissue samples were stained with hematoxylin and eosin (HE). Major steps included: after deparaffination and rehydration, the sections were stained with hematoxylin and eosin, followed by conventional dehydration, and sealing, then observed with conventional optical microscope (Olympus IX71 microscope) and photographed (Fig. 2B). HE staining results showed that there were hepatic cytoplasmic vacuoles in liver tissue sections from mice in high-fat diet group, indicating that high-fat diet could cause fat deposition in liver, while the fat deposition in livers from mice in ES and YH-16 treated groups were significantly lower than that in high-fat diet group without drug administration, with inhibition ratios of 78.9%, and 75.2%, respectively (Fig. 2C). This indicated that ES and YH-16 have a significant inhibitory effect on non-alcoholic fat liver disease.

**Example 3 ES and YH-16 significantly improved insulin resistance and glucose intolerance in mice**

The mice in Example 1 were subjected to an insulin tolerance test 6 hours after completion of administration on day 59. Specific steps included: the tails of mice were cut and blood was collected, basic blood glucose concentrations were measured (Roche hand-held blood glucose meter), and the monitoring time was set to 0 minute. Biosynthetic human insulin (Novolin R, Novo Nordisk) was injected intraperitoneally at 0.5 U/kg, blood samples were taken at 20 min, 40 min, 60 min, 80 min after injection of insulin, and the blood glucose concentrations were measured and a curve was plotted. (Fig. 3A). It was found that after insulin injection, the blood glucose levels of mice in normal diet group quickly reduced over time, while the blood glucose levels of mice in high-fat diet group reduced slowly, indicating that high-fat diet could cause insulin resistance, and ES and YH-16 could significantly alleviate insulin resistance caused by high-fat diet.

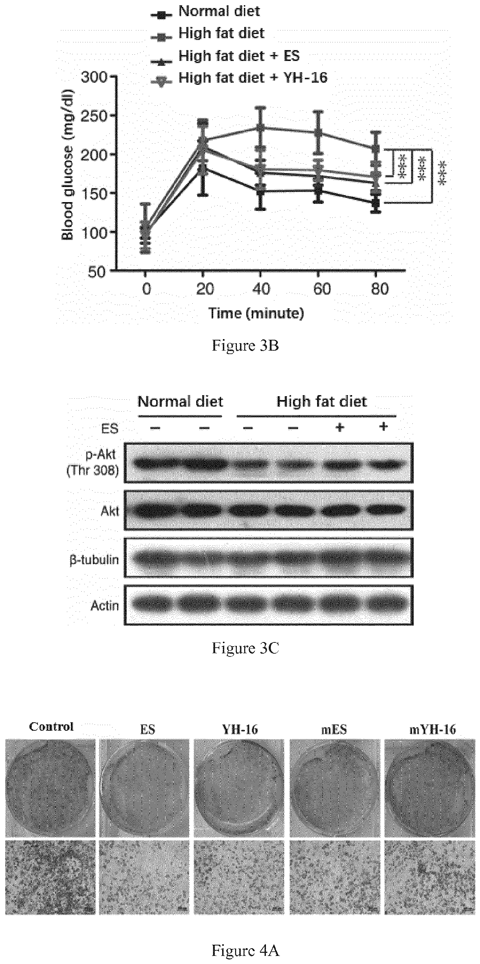

The mice in Example 1, after weighing on day 60, were subjected to starvation overnight and the glucose tolerance test was performed on day 61. Specific steps included: the tails of mice were cut and blood was collected, basic blood glucose concentrations were measured (Roche hand-held blood glucose meter), and the monitoring time was set to 0 minute. The mice were fed by gavage with glucose solution (20mg/ml), at a dose of 1 mg of glucose per gram of body weight of each mouse. Blood samples were taken at 20 min, 40 min, 60 min, 80 min after the gavage with glucose, and the blood glucose levels of mice were measured and a curve was plotted. (Fig. 3B). It was found that after the gavage with glucose, with the passage of time, the blood glucose level of the mice increased rapidly and the recovery rate was slow in high-fat diet group compared to the normal diet group, indicating that high-fat diet could lead to glucose intolerance in mice, while glucose intolerance in mice of ES and YH-16 treated group was significantly improved.

After completion of the glucose tolerance test on day 61, the mice were sacrificed and whole body adipose tissues were isolated, then the phosphorylation levels of Akt in adipose tissues were detected by Western blot (Fig. 3C). The results showed that compared with normal diet group, the phosphorylation level of Akt was lower in high-fat diet group, while the phosphorylation level of Akt in ES treated group was higher than that in high-fat diet group. Akt pathway is an important blood glucose regulatory pathway downstream of insulin. Insulin resistance often accompanies with decreased Akt phosphorylation level. This is consistent with the fact that ES could effectively improve insulin resistance and glucose intolerance.

**Example 4 ES and YH-16 significantly inhibited the differentiation of preadipocytes into adipocytes.**

3T3-L1 preadipocytes in good condition were selected and resuspended in DMEM medium supplemented with 10% FBS, then seeded into six-wells plate, and conventionally incubated at 37°C, 5% $CO_2$ in an incubator. The cells grew for two days, then began to induce differentiation: Step 1. MDI induction medium was added for induction (defining the time as day 1 of cell differentiation); Step 2. two days later, the medium was changed to insulin induction medium, and continued to culture for two more days; Step 3. the medium was changed to DMEM medium supplemented with 10% FBS, and continued to culture until day 8, 3T3-L1 were differentiated into adipocytes. This experiment was divided into 5 groups:

Group 1: control group;

Group 2: ES treated group;

Group 3: YH-16 treated group;

Group 4: mES treated group;

Group 5: mYH-16 treated group.

Among them, drug treated groups were supplemented with 50 μg/ml ES, YH-16, mES or mYH-16 during induction (i.e. day 1 to day 8), control group was added with equal volume of protein buffer. Aforesaid drug supplement and control treatment were carried out at each time when the medium was replaced.

MDI induction medium was prepared by adding 1 μM Dexamethasone, 0.5 mM 3-isobutyl-1-methylxanthine and 10 μg/ml bovine insulin into DMEM medium supplemented with 10% FBS. Insulin induction medium was prepared by adding 10 μg/ml bovine insulin into DMEM medium supplemented with 10% FBS.

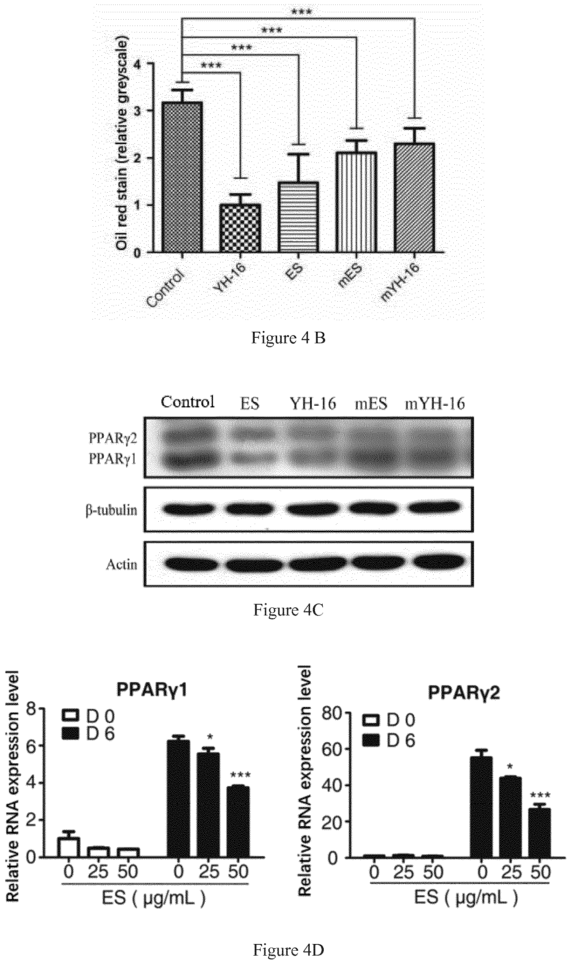

After induction, the medium in the six-well plate was removed, and the cells were fixed and stained with Oil red for 10 minutes. Then the cells were decolorized and rinsed three times with PBS to remove excess dye. Fats in adipocyte could be identified by Oil red, and then stained to red. The six-well plate was photographed with a digital camera, also observed and recorded by photograph with an invert microscope (Olympus IX71 microscope) (Fig. 4A). The results showed that ES, YH-16, mES and mYH-16 all could inhibit the differentiation of preadipocytes into adipocytes (Fig. 4B).

The cells were harvested on day 6 of the induction process, with medium removed. 100 μl 2X SDS electrophoretic loading buffer was added, and the cells were heated at 100°C for 15 minutes. After electrophoresis and film transfer, the expression levels of PPARγ1 and PPARγ2, the central control factors in adipocyte differentiation, in whole cell lysates from each group were detected by immunoblotting (Fig. 4C). It was found that in adipocyte differentiation, ES, YH-16, mES and mYH-16 all could inhibit the protein expression levels of PPARγ1 and PPARγ2, both of which are the central control transcription factors in adipocyte differentiation.

Detection of PPARγ1 and PPARγ2 mRNA expression levels: the total RNA of 3T3-L1 was extracted according to the standard protocol of TRIZOL reagent (purchased from Invitrogen) protocol prior to induction (day 0) and on day 6 of induction. Fermentas reverse transcription kit (RevertAid™ First Stand cDNA Synthesis Kits) was used for reverse transcription according to the standard protocol.

PPARγ1/2, central control factor of adipocyte differentiation, was detected by fluorescence quantitative Real-Time PCR with Stratagene kit (Brilliant II SYBR®Green QRT-PCR Master Mix), MX3000P (purchased from Stratagene) as fluorescence quantitative PCR instrument, SYBR Green as fluorescent dye, with PCR reaction system of 20 μl, and reaction cycles of 40.

PCR procedure: denaturing at 95°C, 10s; annealing and extending at 60°C, 30s; reaction system of 20 μl, reaction cycles of 40; finally keeping at 75°C, 5minutes. GAPDH was used as internal reference. The reaction primers were as follows:

PPARγ1 forward primer (5'-3'): ACAAGATTTGAAAGAAGCGGTGA

PPARγ1 reverse primer (5'-3'): GCTTGATGTCAAAGGAATGCGAAGGA

PPARγ2 forward primer (5'-3'): CGCTGATGCACTGCCTATGAG

PPARγ2 reverse primer (5'-3'): TGGGTCAGCTCTTGTGAATGGAA

GAPDH forward primer (5'-3'): CCAGCCTCGTCCCGTAGACA

GAPDH reverse primer (5'-3'): TGAATTTGCCGTGAGTGGAGTC

Using GAPDH as internal reference, ΔCt values were obtained according to the fluorescence diagram given by the instrument, then relative Δ(ΔCt) values were calculated, and then relative changes in mRNA levels of PPARγ1 and PPARγ2 were calculated (Fig. 4D). It was found that ES could inhibit the mRNA expression level of PPARγ1 and PPARγ2, the central control transcription factors, in adipocyte differentiation.

**Example 5 PEG-modified ES and its variants 003 and 007 (mES, m003, and m007) significantly inhibited weight gain induced by high-fat diet in mice**

A total of 40 healthy C57BL/6 mice (7-week old, male, purchased from Beijing Vital River Laboratory Animal Technology Company) were divided into 5 groups with 8 mice in each group, and treated as follows:

Group 1: normal diet group;

Group 2: high-fat diet group;

Group 3: high-fat diet + mES treated group (drug treated group);

Group 4: high-fat diet + m003 treated group (drug treated group);

Group 5: high-fat diet + m007 treated group (drug treated group).

The diets for each group were the same as in Example 1.

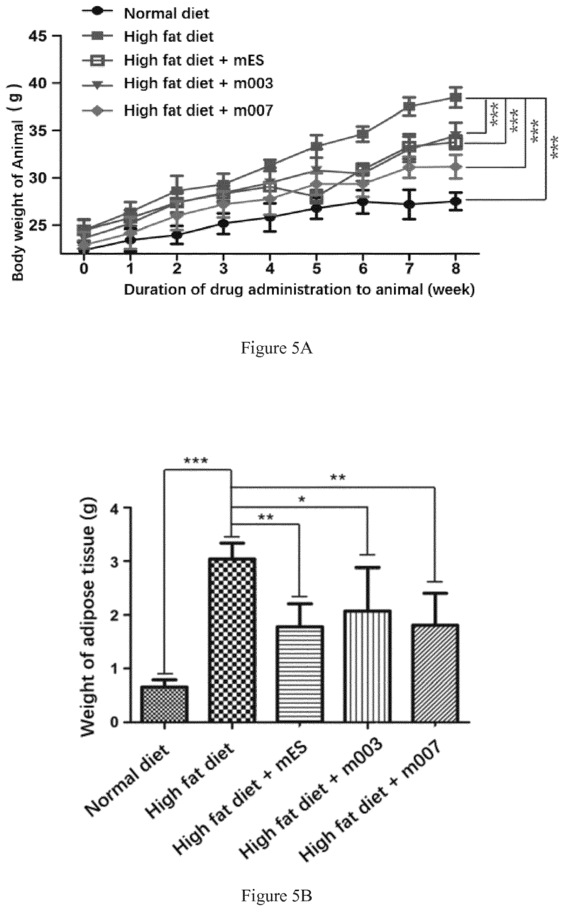

Route of administration: in a period of 8 weeks, group 3, group 4 and group 5 were injected with mES, m003 or m007 (Protgen) via tail vein once a week, at a dose of 50 mg/kg/week, group 2 was injected with equal volume of saline, and group 1 was not injected. The first time of injection was set as week 0, the last administration was carried out on week 7. The mice were weighed once a week, and after the last measurement in week 8 the body weight curves were plotted (Fig. 5A). The results showed that mES, m003, and m007 significantly inhibited weight gain due to high-fat diet, and the inhibition ratios were 33.7%, 22.9%, and 42.9%, respectively (Table 2).

After the last mice body weight measurement in week 8, the mice were sacrificed and whole body adipose tissues were isolated and weighed (Fig. 5B, Table 2). The results showed that the adipose tissue weight of mice in mES, m003, and m007 groups were significantly lower than that in high-fat diet group without drug treatment. The inhibition ratios of mES, m003, and m007 on fat accumulation induced by high-fat diet in mice were 41.4%, 31.9%, and 40.5%, respectively (Table 2).

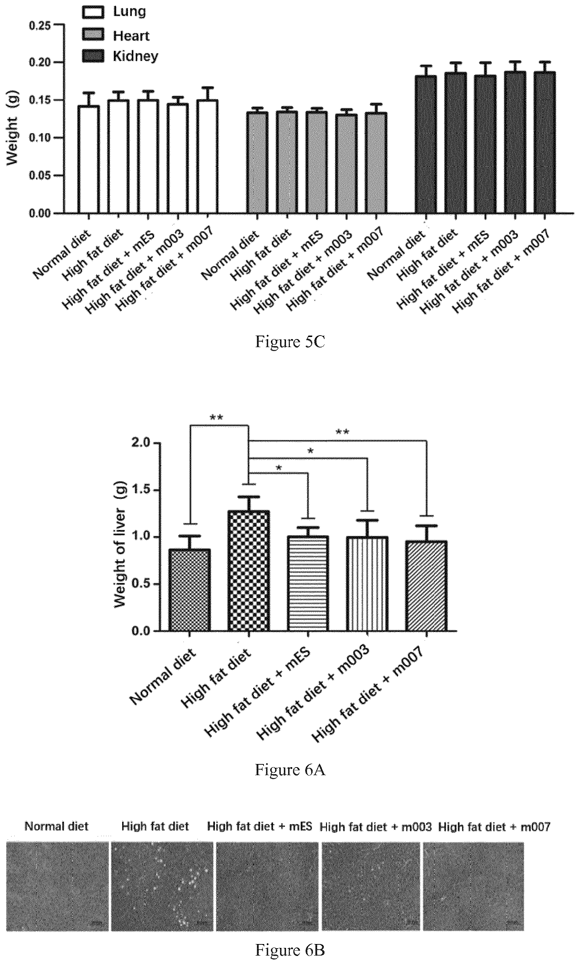

Lungs, heart and kidneys were isolated from mice and weighed (Fig. 5C, Table 2). The results showed that there was no significant difference in the weight of lungs, heart and kidneys among the mice in all five groups, indicating that mES, m003, and m007 had no effect on lungs, heart and kidneys of mice.

**Example 6 PEG-modified ES and its variants 003 and 007 (mES, m003, and m007) significantly inhibited the increase in liver weight and fat deposition induced by high-fat diet in mice**

From the mice in Example 5, after the last mice body weight measurement in week 8, the liver tissues were removed and weighed (Fig. 6A, Table 2). The results show that mES, m003, and m007 inhibited the increase in liver weight induced by high-fat diet in mice, with inhibition ratios of 21.3%, 21.3%, and 25.2%, respectively (Table 2).

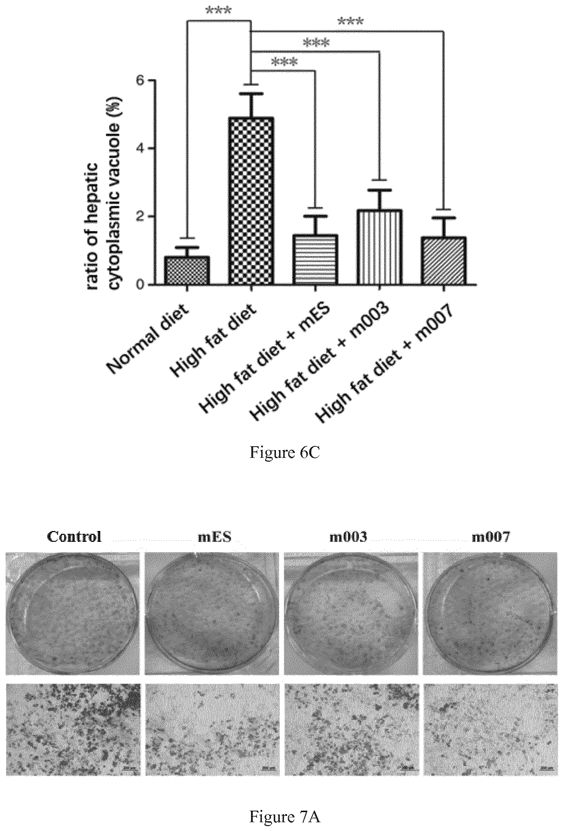

According to the protocol in example 2, the liver tissues were fixed and embedded in paraffin, stained with HE, observed and recorded conventional optical microscope (Olympus IX71 microscope) (Fig. 6B). HE staining results showed that the fat deposition in livers from mice in mES, m003, and m007 treated groups were significantly lower than that in high-fat diet group without drug administration, with the inhibition ratios of 70.6%, 56.1%, and 73.1%, respectively (Fig. 6C). This indicated that mES, m003, and m007 have a significant inhibitory effect on non-alcoholic fatty liver disease.

**Example 7 PEG-modified ES and its variants 003 and 007 (mES, m003, and m007) significantly inhibited the differentiation of preadipocytes into adipocytes**

3T3-L1 preadipocytes were cultured and induced in the same way as in Example 4. The experiment was divided into 4 groups:

Group 1: control group;

Group 2: mES treated group;

Group 3: m003 treated group;

Group 4: m007 treated group.

Among them, drug treated groups were supplemented with extra 50 $\mu$g/ml mES, m003 or m007 during induction (i.e. day 1 to day 8), control group was added with equal volume of protein buffer. Aforesaid drug supplement and control treatment were carried out at each time when the medium was replaced.

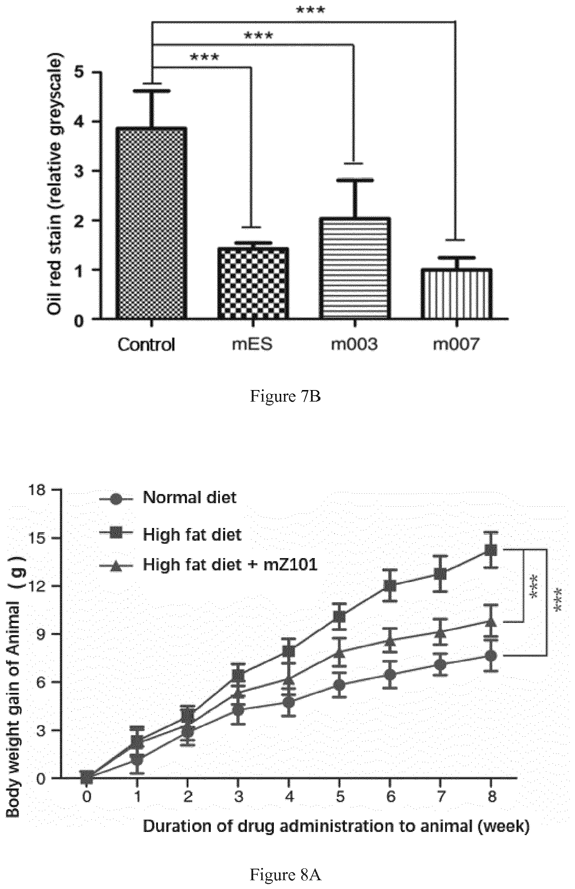

After induction, cells were stained with Oil red according to the experiment method in Example 4. The six-well plate was photographed with a digital camera, also observed and recorded by photograph with an invert microscope (Olympus IX71 microscope) (Fig. 7A). The results showed that mES, m003, and m007 all could directly inhibit the differentiation of preadipocytes into adipocytes, wherein mES and m007 had better inhibition effect than m003 (Fig. 7B). This was consistent with the results of the animal experiment in Example 6, which also explained the reasons why mES and m007 were more effective than m003 in inhibiting weight gain in animals with high-fat diet.

**Example 8 PEG-modified ES variant Z101 (mZ101) significantly inhibited weight gain induced by high-fat diet in mice**

The preparation of experimental mice (8 mice of each group), diet (feedstuff), route of administration, administration cycle and mice body weight measurement were the same as in Example 5. The experiment was grouped as follows:

Group 1: normal diet group;

Group 2: high-fat diet group;

Group 3: high-fat diet + mZ101 treated group (drug treated group).

Wherein the dose was 12 mg/kg/week.

After the last mice body weight measurement in week 8, the body weight curves were plotted (Fig. 8A). The results showed that mZ101 significantly inhibited weight gain due to high-fat diet, and the inhibition ratio was 31 % (Table 3).

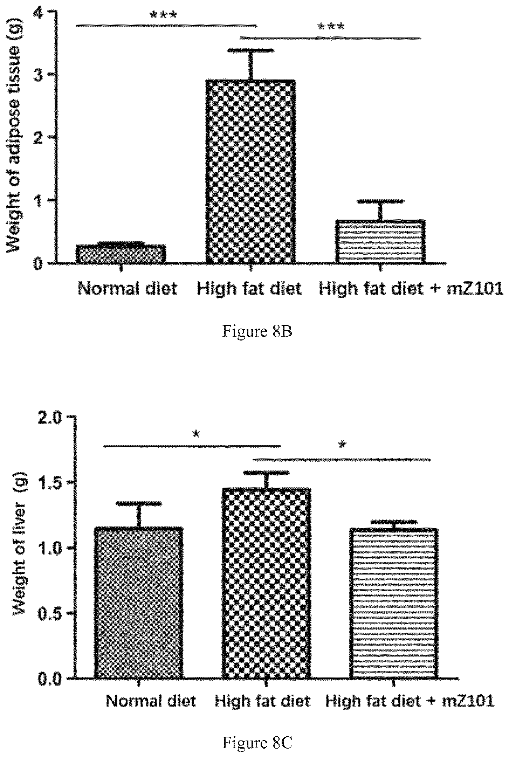

After the last mice body weight measurement in week 8, the mice were sacrificed and whole body adipose tissues were isolated and weighed (Fig. 8B and C, Table 3). The results showed that the adipose tissue weight of mice in mZ101 group was significantly lower than that in high-fat diet group without drug treatment, and the inhibition ratio of fat accumulation was 77.2% (Table 3). mZ101 could also inhibit the increase in mice liver weight, and the inhibition ratio was 21.5% (Table 3).

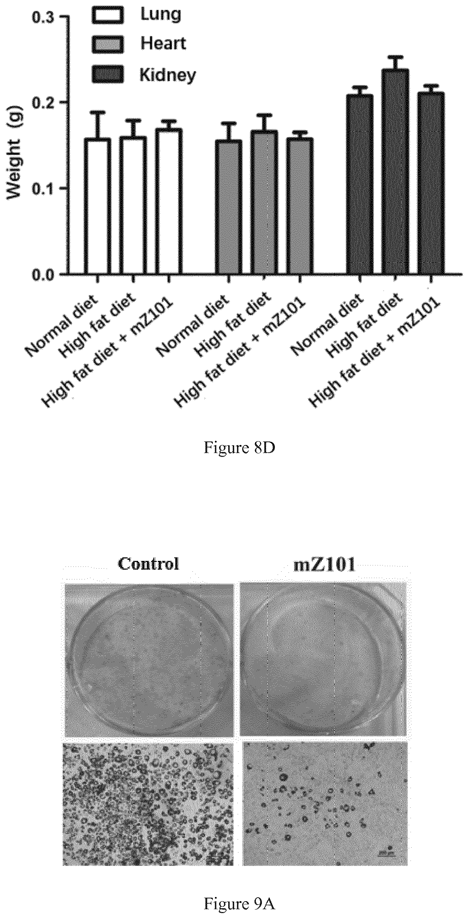

Lungs, heart and kidneys were isolated from mice and weighed (Fig. 8D, Table 3). The results showed that there was no significant difference in weight of mice lungs, heart and kidneys among the three groups, indicating that mZ101 had no effect on lungs, heart and kidneys of mice.

**Example 9 PEG-modified ES variant Z101 (mZ101) significantly inhibited the differentiation of preadipocytes into adipocytes**

3T3-L1 preadipocytes were cultured and induced in the same way as in Example 4. The experiment was divided into 2 groups:

Group 1: control group;

Group 2: mZ101 treated group.

Among them, drug treated group was supplemented with extra 50 $\mu$g/ml mZ101 during induction (i.e. day 1 to day 8), control group was added with equal volume of protein buffer. Aforesaid drug supplement and control treatment were carried out at each time when the medium was replaced.

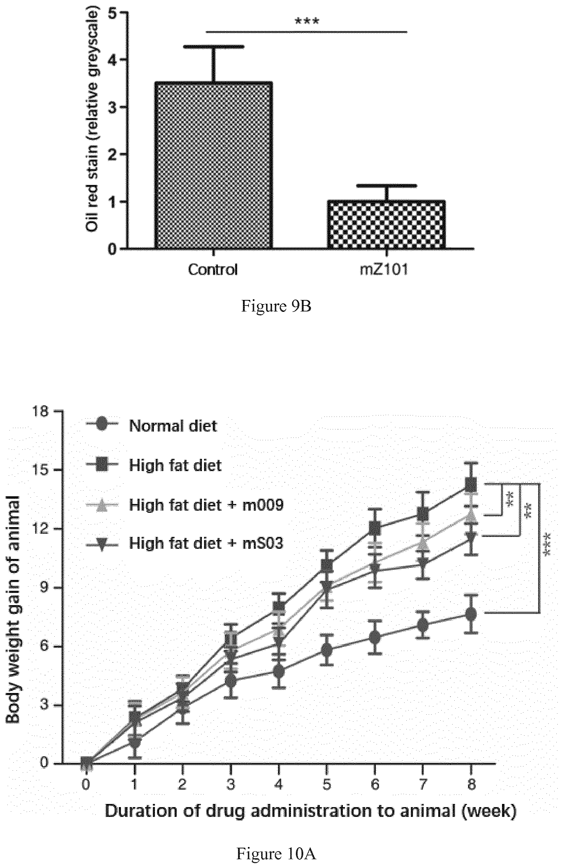

After induction, cells were stained with Oil red according to the experiment method in Example 4. The six-well plate was photographed with a digital camera, also observed and recorded by photograph with an invert microscope (Olympus IX71 microscope) (Fig. 9A). The results showed that mZ101 could directly inhibit the differentiation of preadipocytes into adipocytes (Fig. 9B).

**Example 10 PEG-modified ES variants 009 and S03 (m009 and mS03) significantly inhibited weight gain induced by high-fat diet in mice, and the inhibitory effect of mS03 was better than that of m009**

The preparation of experimental mice (8 mice of each group), diet (feedstuff), route of administration, administration cycle and mice body weight measurement were the same as in Example 5. The experiment was grouped as follows:

Group 1: normal diet group;

Group 2: high-fat diet group;

Group 3: high-fat diet + m009 treated group (drug treated group);

Group 4: high-fat diet + mS03 treated group (drug treated group).

Wherein the dose was 12 mg/kg/week.

After the last mice body weight measurement in week 8, the body weight curves were plotted (Fig. 10A). The results showed that m009 and mS03 significantly inhibited weight gain due to high-fat diet, and the inhibition ratios were 10.6%, and 19.0%, respectively (Table 4).

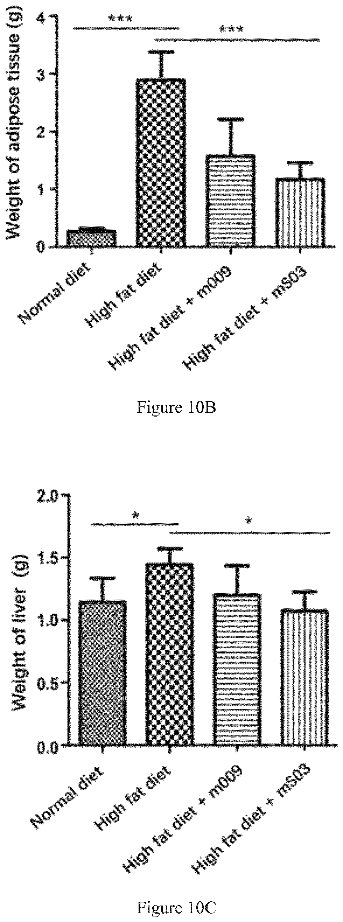

After the last mice body weight measurement in week 8, the mice were sacrificed and whole body adipose tissues were isolated and weighed (Fig. 10B and C, Table 4). The results showed that the weight of adipose tissues of mice in m009 and mS03 groups was significantly lower than that in high-fat diet group without drug treatment, and the inhibition ratios of fat accumulation were 45.7%, and 59.5%, respectively (Table 4). m009 and mS03 could also inhibit the increase in mice liver weight, and the inhibition ratios were 16.7%, and 25.7%, respectively (Table 4).

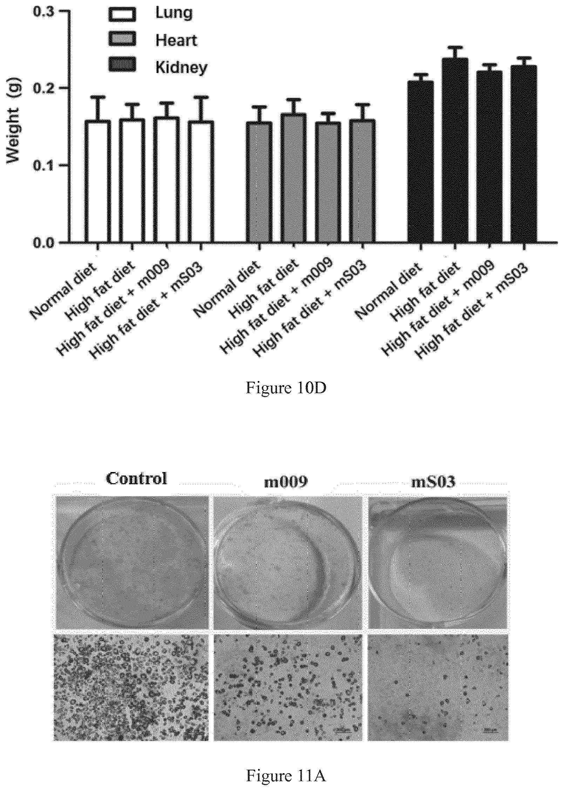

Lungs, heart and kidneys were isolated from mice and weighed (Fig. 10D, Table 4). The results showed that there was no significant difference in weight of mice lungs, heart and kidneys among the four groups, indicating that m009 and mS03 had no effect on lungs, heart and kidneys of mice.

**Example 11 PEG-modified ES variants 009 and S03 (m009 and mS03) significantly inhibited the differentiation of preadipocytes into adipocytes**

3T3-L1 preadipocytes were cultured and induced in the same way as in Example 4. The experiment was divided into 3 groups:

Group 1: control group;

Group 2: m009 treated group;

Group 3: mS03 treated group.

Among them, drug treated groups were supplemented with extra 50 $\mu$g/ml m009 or mS03 during induction (i.e. day 1 to day 8), control group was added with equal volume of protein buffer. Aforesaid drug supplement and control treatment were carried out at each time when the medium was replaced.

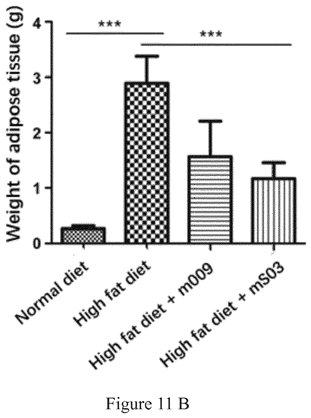

After induction, cells were stained with Oil red according to the experiment method in Example 4. The six-well plate was photographed with a digital camera, also observed and recorded by photograph with an invert microscope (Olympus IX71 microscope) (Fig. 11A). The results showed that both m009 and mS03 could directly inhibit the differentiation of preadipocytes into adipocytes, wherein the mS03 had better inhibition effect than m009 (Fig. 11B). This was consistent with the results of the animal experiment in Example 9, which also explained the reasons why mS03 was more effective than m009 in inhibiting weight gain in animals with high-fat diet.

**Example 12 PEG-modified ES variants 36 and 249 (m36 and m249) significantly inhibited weight gain induced by high-fat diet in mice**

The preparation of experimental mice (8 mice of each group), diet (feed), route of administration, administration cycle and mice body weight measurement were the same as in Example 5. The experiment was grouped as follows:

Group 1: normal diet group;

Group 2: high-fat diet group;

Group 3: high-fat diet + m36 (6 mg/kg/week) treated group (drug treated group);

Group 4: high-fat diet + m36 (12 mg/kg/week) treated group (drug treated group);

Group 5: high-fat diet + m249 (6 mg/kg/week) treated group (drug treated group);

Group 6: high-fat diet + m249 (12 mg/kg/week) treated group (drug treated group).

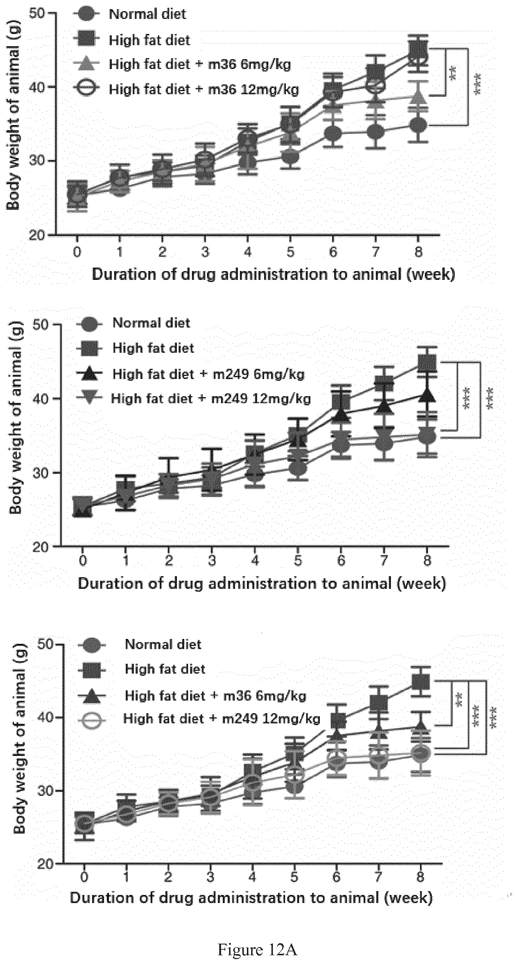

After the last mice body weight measurement in week 8, the body weight curves were plotted (Fig. 12A). The results showed that low-dose m36 (6 mg/kg/week) and high-dose m249 (12 mg/kg/week) significantly inhibited weight gain due to high-fat diet, and the inhibition ratios were 30.3%, and 50.3%, respectively (Table 5).

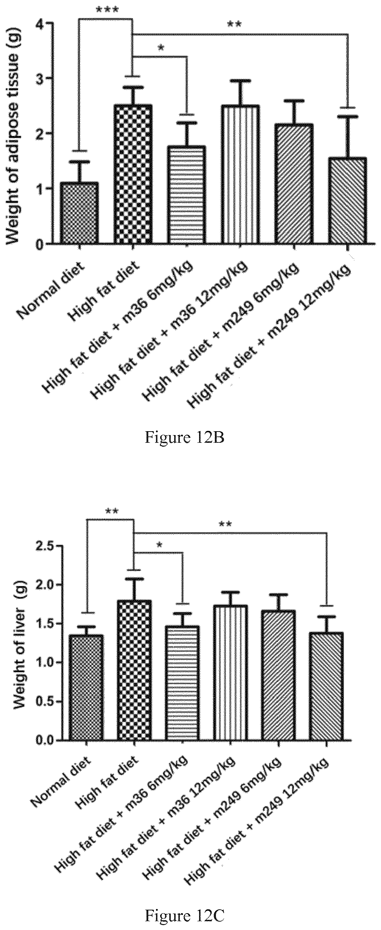

After the last mice body weight measurement in week 8, the mice were sacrificed and whole body adipose tissues were isolated and weighed (Fig. 12B and C, Table 5). The results showed that the weight of adipose tissues of mice in low-dose m36 (6 mg/kg/week) and high-dose m249 (12 mg/kg/week) groups was significantly lower than that in high-fat diet group without drug treatment, and the inhibition ratios of low-dose m36 (6 mg/kg/week) and high-dose m249 (12 mg/kg/week) on fat accumulation induced by high-fat diet in mice were 30%, and 38.4%, respectively (Table 5). Low-dose m36 (6 mg/kg/week) and high-dose m249 (12 mg/kg/week) could also inhibit the increase in mice liver weight, and the inhibition ratios were 18.4%, and 22.9%, respectively (Table 5).

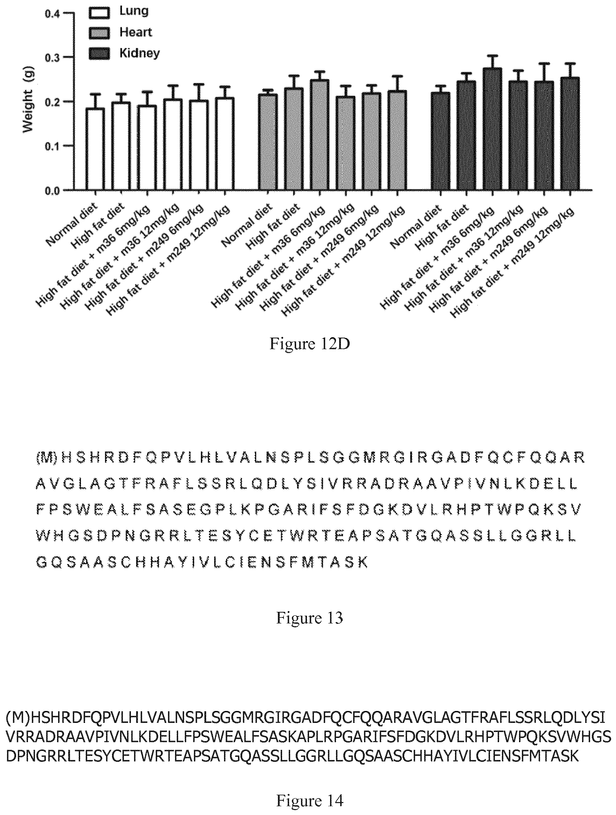

Lungs, heart and kidneys were isolated from mice and weighed (Fig. 12D). The results showed that there was no significant difference in the weight of lungs, heart and kidneys among the six groups, indicating that m36 and m249 had no effect on lungs, heart and kidneys of mice.

Table 1

|  | Group of normal diet | Group of high-fat diet | Group of high-fat diet + ES | Group of high-fat diet + YH-16 |
|---|---|---|---|---|
| Mice body weight before administration (g) | $22.4 \pm 0.83$ | $23.5 \pm 0.88$ | $23.4 \pm 1.25$ | $23.5 \pm 1.04$ |
| Mice body weight after administration (g) | $28.1 \pm 0.89$ | $37.9 \pm 1.35$ | $32.4 \pm 0.83$ | $33.5 \pm 0.97$ |
| Mice body weight gain (g) | $5.7 \pm 0.79$ | $14.4 \pm 2.51$ | $9.0 \pm 1.84$ | $10.00 \pm 1.61$ |
| Mice adipose tissue weight after administration (g) | $0.66 \pm 0.13$ | $2.89 \pm 0.59$ | $1.51 \pm 0.67$ | $1.67 \pm 0.48$ |
| Mice liver weight after administration (g) | $0.86 \pm 0.15$ | $1.22 \pm 0.18$ | $0.93 \pm 0.14$ | $0.97 \pm 0.12$ |
| Mice heart weight after administration (g) | $0.133 \pm 0.006$ | $0.131 \pm 0.007$ | $0.132 \pm 0.008$ | $0.138 \pm 0.009$ |
| Mice lung weight after administration (g) | $0.141 \pm 0.018$ | $0.142 \pm 0.008$ | $0.142 \pm 0.006$ | $0.147 \pm 0.012$ |
| Mice kidney weight after administration (g) | $0.181 \pm 0.014$ | $0.18 \pm 0.01$ | $0.189 \pm 0.015$ | $0.191 \pm 0.010$ |

Table 2

|  | Group of normal diet | Group of high-fat diet | Group of high-fat diet + mES | Group of high-fat diet + m003 | Group of high-fat diet + m007 |
|---|---|---|---|---|---|
| Mice body weight before administration (g) | $22.4 \pm 0.83$ | $24.5 \pm 1.06$ | $24.5 \pm 1.14$ | $23.6 \pm 1.09$ | $22.9 \pm 1.38$ |
| Mice body weight after administration (g) | $27.5 \pm 0.94$ | $38.5 \pm 1.06$ | $33.8 \pm 0.72$ | $34.4 \pm 1.38$ | $31.2 \pm 1.25$ |
| Mice body weight gain (g) | $5.1 \pm 0.87$ | $14.0 \pm 0.82$ | $9.28 \pm 0.91$ | $10.8 \pm 1.95$ | $7.99 \pm 0.95$ |
| Mice adipose tissue weight after administration (g) | $0.66 \pm 0.13$ | $3.04 \pm 0.30$ | $1.78 \pm 0.43$ | $1.81 \pm 0.59$ | $2.07 \pm 0.81$ |
| Mice liver weight after administration (g) | $0.86 \pm 0.15$ | $1.27 \pm 0.16$ | $1.00 \pm 0.10$ | $0.95 \pm 0.18$ | $1.00 \pm 0.18$ |
| Mice heart weight after administration (g) | $0.14 \pm 0.020$ | $0.15 \pm 0.008$ | $0.15 \pm 0.012$ | $0.15 \pm 0.017$ | $0.14 \pm 0.010$ |
| Mice lung weight after administration (g) | $0.133 \pm 0.006$ | $0.134 \pm 0.006$ | $0.134 \pm 0.005$ | $0.132 \pm 0.012$ | $0.130 \pm 0.007$ |
| Mice kidney weight after administration (g) | $0.181 \pm 0.014$ | $0.185 \pm 0.014$ | $0.181 \pm 0.018$ | $0.186 \pm 0.014$ | $0.186 \pm 0.014$ |

Table 3

|  | Group of normal diet | Group of high-fat diet | Group of high-fat diet + mZ101 |
|---|---|---|---|
| Mice body weight before administration (g) | 20.9 ± 0.40 | 21.1 ± 0.42 | 20.7 ± 0.31 |
| Mice body weight after administration (g) | 28.5 ± 0.75 | 35.3 ± 1.75 | 30.6 ± 2.21 |
| Mice body weight gain (g) | 7.6 ± 0.97 | 14.2 ± 1.09 | 9.8 ± 0.98 |
| Mice adipose tissue weight after administration (g) | 0.26 ± 0.05 | 2.89 ± 0.49 | 0.66 ± 0.32 |
| Mice liver weight after administration (g) | 1.14 ± 0.19 | 1.44 ± 0.13 | 1.13 ± 0.06 |
| Mice heart weight after administration (g) | 0.145 ± 0.018 | 0.163 ± 0.014 | 0.155 ± 0.007 |
| Mice lung weight after administration (g) | 0.16 ± 0.031 | 0.16 ± 0.019 | 0.17 ± 0.010 |
| Mice kidney weight after administration (g) | 0.21 ± 0.010 | 0.24 ± 0.015 | 0.21 ± 0.009 |

Table 4 (Figure 11)

|  | Group of normal diet | Group of high-fat diet | Group of high-fat diet + m009 | Group of high-fat diet + mS03 |
|---|---|---|---|---|
| Mice body weight before administration (g) | 20.9 ± 0.40 | 21.1 ± 0.42 | 20.8 ± 0.35 | 20.9 ± 0.44 |
| Mice body weight after administration (g) | 28.5 ± 0.75 | 35.3 ± 1.75 | 33.5 ± 1.80 | 32.4 ± 2.35 |
| Mice body weight gain (g) | 7.6 ± 0.97 | 14.2 ± 1.09 | 12.7 ± 1.03 | 11.5 ± 0.80 |
| Mice adipose tissue weight after administration (g) | 0.26 ± 0.05 | 2.89 ± 0.49 | 1.57 ± 0.64 | 1.17 ± 0.29 |
| Mice liver weight after administration (g) | 1.14 ± 0.19 | 1.44 ± 0.13 | 1.20 ± 0.24 | 1.07 ± 0.15 |
| Mice heart weight after administration (g) | 0.145 ± 0.018 | 0.163 ± 0.014 | 0.156 ± 0.011 | 0.159 ± 0.020 |
| Mice lung weight after administration (g) | 0.16 ± 0.031 | 0.16 ± 0.019 | 0.16 ± 0.019 | 0.16 ± 0.032 |
| Mice kidney weight after administration (g) | 0.21 ± 0.010 | 0.24 ± 0.015 | 0.22 ± 0.010 | 0.23 ± 0.011 |

Table 5 (Figure 12)

|  | Group of normal diet | Group of high-fat diet | Group of high-fat diet + 6mg/kg/week of m36 | Group of high-fat diet + 12mg/kg/week of m36 | Group of high-fat diet + 6mg/kg/week of m249 | Group of high-fat diet + 12mg/kg/week of m249 |
|---|---|---|---|---|---|---|
| Mice body weight before administration (g) | 25.4 ± 1.06 | 25.4 ± 1.24 | 25.1 ± 1.87 | 25.5 ± 1.71 | 25.0 ± 0.64 | 25.5 ± 0.98 |
| Mice body weight after administration (g) | 34.9 ± 2.31 | 44.9 ± 2.02 | 38.7 ± 2.02 | 44.1 ± 2.04 | 40.6 ± 3.03 | 35.2 ± 3.03 |

(continued)

|  | Group of normal diet | Group of high-fat diet | Group of high-fat diet + 6mg/kg/week of m36 | Group of high-fat diet + 12mg/kg/week of m36 | Group of high-fat diet + 6mg/kg/week of m249 | Group of high-fat diet + 12mg/kg/week of m249 |
|---|---|---|---|---|---|---|
| Mice body weight gain (g) | 9.5 ± 2.64 | 19.5 ± 2.43 | 13.6 ± 3.73 | 18.6 ± 3.06 | 15.6 ± 2.48 | 9.7 ± 2.42 |
| Mice adipose tissue weight after administration (g) | 1.09 ± 0.39 | 2.50 ± 0.33 | 1.75 ± 0.44 | 2.49 ± 0.46 | 2.15 ± 0.44 | 1.54 ± 0.76 |
| Mice liver weight after administration (g) | 1.34 ± 0.12 | 1.79 ± 0.29 | 1.46 ± 0.17 | 1.73 ± 0.18 | 1.66 ± 0.21 | 1.38 ± 0.21 |
| Mice heart weight after administration (g) | 0.21 ± 0.010 | 0.23 ± 0.029 | 0.25 ± 0.020 | 0.21 ± 0.024 | 0.22 ± 0.018 | 0.22 ± 0.034 |
| Mice lung weight after administration (g) | 0.183 ± 0.033 | 0.197 ± 0.020 | 0.190 ± 0.032 | 0.204 ± 0.031 | 0.201 ± 0.037 | 0.208 ± 0.025 |
| Mice kidney weight after administration (g) | 0.22 ± 0.015 | 0.24 ± 0.019 | 0.27 ± 0.029 | 0.24 ± 0.025 | 0.24 ± 0.042 | 0.25 ± 0.033 |

The names and the corresponding amino acid sequences of ES and its mutants according to the present invention are as follows:

ES (SEQ ID NO:1)

(M)HSHRDFQPVLHLVALNSPLSGGMRGIRGADFQCFQQARAVGLAGTFRAFLSSRLQDLYSIVRRADRAAVPIVNLKDELLFPSWEALFSGSEGPLKPGARIFSFDGKDVLRHPTWPQKSVW HGSDPNGRRLTESYCETWRTEAPSATGQASSLLGGRLLGQSAASCHHAYIVLCIENSFMTASK

YH16 (SEQ ID NO:2)

(M)GGSHHHHHHSHRDFQPVLHLVALNSPLSGGMRGIRGADFQCFQQARAVGLAGTFRAFLSSRLQDLYSIVRRADRAAVPIVNLKDELLFPSWEALFSGSEGPLKPGARIFSFDGKDVLRHPT WPQKSVWHGSDPNGRRLTESYCETWRTEAPSATGQASSLLGGRLLGQSAASCHHAYIVLCIENSFMTASK

003 (SEQ ID NO:3)

(M)HSHRDFQPVLHLVALNSPLSGGMRGIRGADFQCFQQARAVGLAGTFRAFLSSRLQDLYSIVRRADRAAVPIVNLKDELLFPSWEALFSASEGPLKPGARIFSFDGKDVLRHPTWPQKSVW HGSDPNGRRLTESYCETWRTEAPSATGQASSLLGGRLLGQSAASCHHAYIVLCIENSFMTASK

007 (SEQ ID NO:4)

(M)HSHRDFQPVLHLVALNSPLSGGMRGIRGADFQCFQQARAVGLAGTFRAFLSSRLQDLYSIVRRADRAAVPIVNLKDELLFPSWEALFSASKAPLQPGARIFSFDGKDVLRHPTWPQKSVW HGSDPNGRRLTESYCETWRTEAPSATGQASSLLGGRLLGQSAASCHHAYIVLCIENSFMTASK

Z101 (SEQ ID NO:5)

(M)HSHRDFQPVLHLVALNSPLSGGMRGIRGADFQCFQQARAVGLAGTFRAFLSSRLQDLYSIVRRADRAAVPIVNLKDELLFPSWEALFSSEGPLKPGARIFSFDGRDVLRHPTWPQRSVWH GSDPNGRRLTESYCETWRTEAPSATGQASSLLGGRLLGQSAASCHHAYIVLCIENSFMTASR

009 (SEQ ID NO:6)

(M)HSHQDFQPVLHLVALNSPLSGGMRGIRGADFQCFQQARAVGLAGTFRAFLSSRLQDLYSIVRRADRAAVPIVNLKDELLFPSWEALFSSEGPLQPGARIFSFDGKDVLRHPTWPQKSVWH GSDPNGRRLTESYCETWRTEAPSATGQASSLLGGRLLGQSAASCHHAYIVLCIENSFMTASK

### S03 (SEQ ID NO:7)

(M)DFQPVLHLVALNSPLSGGMRGIRGADFQCFQQARAVGLAGTFRAFLSSRLQDLYSIVRRADRAAVPIVNLKDELLFPSWEALFSGESGAGKTPGARIFSFDGKDVLRHPTWPQKSVWHGS DPNGRRLTESYCETWRTEAPSATGQASSLLGGRLLGQSAASCHHAYIVLCIENSFMTASK

### 36 (SEQ ID NO:8)

(M)RDFQPVLHLVALNSPLSGGMRGIRGADFQCFQQARQVGLAGTFRAFLSSRLQDLYSIVRRADRAAVPIVNLKDELLFPSWEALFSSEGPLKPGARIFSFDGKDVLRHPTWPQKSVWHGSD PNGRRLTESYCETWRTEAPSATGQASSLLGGRLLGQSAASCHHAYIVLCIENSFMTASK

### 249 (SEQ ID NO:9)

(M)RDFQPVLHLVALNSPLSGGMRGIRGADFQCFQQARAVGLAGTFRAFLSSRLQDLYSIVRRADRGSVPIVNLKDEVLSPSWDSLFSGSQGQLQPGARIFSFDGRDILQDSAWPQKSVWHGS DAKGRRLPESYCEAWRTDERGTSGQASSLLSGRLLEQKAASCHNSYIVLCIENSFMTASK

### 381 (SEQ ID NO:10)

(M)HVHQDFQPALHLVALNTPLSGGMRGIRGADFQCFQQARQVGLAGTFRAFLSSRLQDLYSIVRRADRTAVPIVNLRDEVLFSNWEALFTGSEAPLRAGARIFSFDGRDVLRHPTWPQKSV WHGSDPNGRRLTESYCETWRTEAPSATGQASSLLAGRLLEQKAAGCHNAFIVLCIENSFMTSSSK

### 57 (SEQ ID NO:11)

(M)HTHQDFHPVLHLVALNTPLSGGMRGIRGADFQCFQQARAVGLSGTFRAFLSSRLQDLYSIVRRADRAAVPIVNLKDELLFPSWEALFSGESGAGKTGGARIFSFDGRDVLRHPAWPQKSV WHGSDPSGRRLTESYCETWRTDSRAATGQASSLLAGRLLEQKAAGCHNAFIVLCIENSFMTSSSK

### 114 (SEQ ID NO:12)

(M)HSHRDFQPVLHLVALNSPLSGGMRGIRGADFQCFQQARAVGLAGTFRAFLSSRLQDLYSIVRRADRAAVPIVNLKDELLFPSWEALFSGSEGPLKPGARIFSFDGRDVLRHPTWPQKSVW HGSDPSGHRLTESYCETWRTDSRAATGQASSLLGGRLLGQSAASCHHAYIVLCIANSFMTASK

### 124 (SEQ ID NO:13)

(M)DFQPVLHLVALNSPLSGGMRGIRGADFQCFQQARAVGLAGTFRAFLSSRLQDLYSIVRRADRAAVPIVNLKDELLFPSWEALFSGSEGPLRPGARIFSFDGKDVLRHPTLPQKSVWHGSDP SGRRLTESYCETWRTDSRAATGQASSLLGGRLLGQSAASCHHAYIVLCIENSFMTASK

### 125 (SEQ ID NO:14)

(M)DFQPVLHLVALNSPLSGGMRGIRGADFQCFQQARAVGLAGTFRAFLSSRLQDLYSIVRRADRAAVPIVNLKDELLFPSWEALFSGSEGPLRPGARIFSFDGKDVLRHPTLPQKSVWHGSDP SGRRLTESYCETWRTDSRAATGQASSLLGGRLLGQSAASCHHAYIVLCIENSFMTASKK

### 160 (SEQ ID NO:15)

(M)HTHQDFHPVLHLVALNTPLSGGMRGIRGADFQCFQQARAVGLAGTFRAFLSSRLQDLYSIVRRADRAAVPIVNLKDELLFPSWEALFSGSEGPLKPGARIFSFDGRDILQDSAWPQKSVW HGSDPNGRRLTESYCETWRTEAPSATGQASSLSSGKLLEQSVSSCQHAFVVLCIENSFMTAAKK

### 119 (SEQ ID NO:16)

(M)HTHTSGPGLHLIALNSPQVGNMRGIRGADFQCFQQARAVGLAGTFRAFLSSRLQDLYSIVRRADRSSVPIVNLKDEVLSPSWDSLFSVSQGQLQPGARIFSFDGRDILQDSAWPQKSVWH GSDPNGRRLTESYCETWRTEAPSATGQASSLLGGRLLGQSAASCHHAYIVLCIENSFMTASK

### 163 (SEQ ID NO:17)

(M)TPTWYPRMLRVAALNEPSTGDLQGIRGADFQCFQQARAVGLSGTFRAFLSSRLQDLYSIVRRADRAAVPIVNLKDEVLSPSWDSLFSGSQGQLQPGARIFSFDGKDVLRHPTWPQKSVWH GSDPSGRRLMESYCETWRTETTGATGQASSLLGGRLLGQSAASCHHAYIVLCIENSFMTNNRK

ÐòÁÐ±í

<110> Çå»ª´óÑ§
±±¾©ÆÕÂÞ¼ªÉúÎï¿Æ¼¼•¢Õ¹ÓÐÏÞ¹«Ë¾

<120> Ò»ÖÖÒÖÖÆÖ¬•¾Ï¸°û•Ö»¯ÍÒÈµº·ËØÄÍÊÜµÄÒ©Îï

<130> 0049PCT

<140> PCT/CN2015/093726
<141> 2015-11-03

<160> 17

<170> PatentIn version 3.5

<210> 1
<211> 184
<212> PRT
<213> Homo sapiens

<400> 1

Met His Ser His Arg Asp Phe Gln Pro Val Leu His Leu Val Ala Leu
1               5                   10                  15

Asn Ser Pro Leu Ser Gly Gly Met Arg Gly Ile Arg Gly Ala Asp Phe
            20                  25                  30

Gln Cys Phe Gln Gln Ala Arg Ala Val Gly Leu Ala Gly Thr Phe Arg
            35                  40                  45

Ala Phe Leu Ser Ser Arg Leu Gln Asp Leu Tyr Ser Ile Val Arg Arg
            50                  55                  60

Ala Asp Arg Ala Ala Val Pro Ile Val Asn Leu Lys Asp Glu Leu Leu
65                  70                  75                  80

Phe Pro Ser Trp Glu Ala Leu Phe Ser Gly Ser Glu Gly Pro Leu Lys
                85                  90                  95

Pro Gly Ala Arg Ile Phe Ser Phe Asp Gly Lys Asp Val Leu Arg His
                100                 105                 110

Pro Thr Trp Pro Gln Lys Ser Val Trp His Gly Ser Asp Pro Asn Gly
                115                 120                 125

Arg Arg Leu Thr Glu Ser Tyr Cys Glu Thr Trp Arg Thr Glu Ala Pro
            130                 135                 140

Ser Ala Thr Gly Gln Ala Ser Ser Leu Leu Gly Gly Arg Leu Leu Gly
145                 150                 155                 160

```
Gln Ser Ala Ala Ser Cys His His Ala Tyr Ile Val Leu Cys Ile Glu
            165                 170                 175

Asn Ser Phe Met Thr Ala Ser Lys
            180


<210>   2
<211>   192
<212>   PRT
<213>   ËË¹¤ÐòÁÐ

<220>
<223>   µ°°×ÖÊ±äÌå

<400>   2

Met Gly Gly Ser His His His His His His Ser His Arg Asp Phe Gln
1               5                   10                  15

Pro Val Leu His Leu Val Ala Leu Asn Ser Pro Leu Ser Gly Gly Met
            20                  25                  30

Arg Gly Ile Arg Gly Ala Asp Phe Gln Cys Phe Gln Gln Ala Arg Ala
            35                  40                  45

Val Gly Leu Ala Gly Thr Phe Arg Ala Phe Leu Ser Ser Arg Leu Gln
            50                  55                  60

Asp Leu Tyr Ser Ile Val Arg Arg Ala Asp Arg Ala Ala Val Pro Ile
65                  70                  75                  80

Val Asn Leu Lys Asp Glu Leu Leu Phe Pro Ser Trp Glu Ala Leu Phe
                85                  90                  95

Ser Gly Ser Glu Gly Pro Leu Lys Pro Gly Ala Arg Ile Phe Ser Phe
                100                 105                 110

Asp Gly Lys Asp Val Leu Arg His Pro Thr Trp Pro Gln Lys Ser Val
            115                 120                 125

Trp His Gly Ser Asp Pro Asn Gly Arg Arg Leu Thr Glu Ser Tyr Cys
    130                 135                 140

Glu Thr Trp Arg Thr Glu Ala Pro Ser Ala Thr Gly Gln Ala Ser Ser
145                 150                 155                 160

Leu Leu Gly Gly Arg Leu Leu Gly Gln Ser Ala Ala Ser Cys His His
                165                 170                 175

Ala Tyr Ile Val Leu Cys Ile Glu Asn Ser Phe Met Thr Ala Ser Lys
```

```
                    180                    185                    190


<210>   3
<211>   184
<212>   PRT
<213>   ÈË¹¤ÐòÁÐ

<220>
<223>   µ°°×ÖÊ±äÌå

<400>   3

Met His Ser His Arg Asp Phe Gln Pro Val Leu His Leu Val Ala Leu
1               5                   10                  15


Asn Ser Pro Leu Ser Gly Gly Met Arg Gly Ile Arg Gly Ala Asp Phe
            20                  25                  30


Gln Cys Phe Gln Gln Ala Arg Ala Val Gly Leu Ala Gly Thr Phe Arg
        35                  40                  45


Ala Phe Leu Ser Ser Arg Leu Gln Asp Leu Tyr Ser Ile Val Arg Arg
    50                  55                  60


Ala Asp Arg Ala Ala Val Pro Ile Val Asn Leu Lys Asp Glu Leu Leu
65                  70                  75                  80


Phe Pro Ser Trp Glu Ala Leu Phe Ser Ala Ser Glu Gly Pro Leu Lys
                85                  90                  95


Pro Gly Ala Arg Ile Phe Ser Phe Asp Gly Lys Asp Val Leu Arg His
            100                 105                 110


Pro Thr Trp Pro Gln Lys Ser Val Trp His Gly Ser Asp Pro Asn Gly
            115                 120                 125


Arg Arg Leu Thr Glu Ser Tyr Cys Glu Thr Trp Arg Thr Glu Ala Pro
            130                 135                 140


Ser Ala Thr Gly Gln Ala Ser Ser Leu Leu Gly Gly Arg Leu Leu Gly
145                 150                 155                 160


Gln Ser Ala Ala Ser Cys His His Ala Tyr Ile Val Leu Cys Ile Glu
                165                 170                 175


Asn Ser Phe Met Thr Ala Ser Lys
                180


<210>   4
<211>   184
```

<210>    PRT
<213>    ËË¹¤ÐòÁÐ

<220>
<223>    µ°°×ÖÊ±äÌå

<400>    4

Met His Ser His Arg Asp Phe Gln Pro Val Leu His Leu Val Ala Leu
1                   5                   10                  15

Asn Ser Pro Leu Ser Gly Gly Met Arg Gly Ile Arg Gly Ala Asp Phe
            20                  25                  30

Gln Cys Phe Gln Gln Ala Arg Ala Val Gly Leu Ala Gly Thr Phe Arg
            35                  40                  45

Ala Phe Leu Ser Ser Arg Leu Gln Asp Leu Tyr Ser Ile Val Arg Arg
            50                  55                  60

Ala Asp Arg Ala Ala Val Pro Ile Val Asn Leu Lys Asp Glu Leu Leu
65                  70                  75                  80

Phe Pro Ser Trp Glu Ala Leu Phe Ser Ala Ser Lys Ala Pro Leu Gln
                85                  90                  95

Pro Gly Ala Arg Ile Phe Ser Phe Asp Gly Lys Asp Val Leu Arg His
            100                 105                 110

Pro Thr Trp Pro Gln Lys Ser Val Trp His Gly Ser Asp Pro Asn Gly
            115                 120                 125

Arg Arg Leu Thr Glu Ser Tyr Cys Glu Thr Trp Arg Thr Glu Ala Pro
            130                 135                 140

Ser Ala Thr Gly Gln Ala Ser Ser Leu Leu Gly Gly Arg Leu Leu Gly
145                 150                 155                 160

Gln Ser Ala Ala Ser Cys His His Ala Tyr Ile Val Leu Cys Ile Glu
                165                 170                 175

Asn Ser Phe Met Thr Ala Ser Lys
                180

<210>    5
<211>    183
<212>    PRT
<213>    ËË¹¤ÐòÁÐ

<220>
<223>    µ°°×ÖÊ±äÌå

<400>   5

Met His Ser His Arg Asp Phe Gln Pro Val Leu His Leu Val Ala Leu
1               5               10                  15

Asn Ser Pro Leu Ser Gly Gly Met Arg Gly Ile Arg Gly Ala Asp Phe
            20                  25                  30

Gln Cys Phe Gln Gln Ala Arg Ala Val Gly Leu Ala Gly Thr Phe Arg
        35                  40                  45

Ala Phe Leu Ser Ser Arg Leu Gln Asp Leu Tyr Ser Ile Val Arg Arg
        50                  55                  60

Ala Asp Arg Ala Ala Val Pro Ile Val Asn Leu Lys Asp Glu Leu Leu
65                  70                  75                  80

Phe Pro Ser Trp Glu Ala Leu Phe Ser Ser Glu Gly Pro Leu Lys Pro
                85                  90                  95

Gly Ala Arg Ile Phe Ser Phe Asp Gly Arg Asp Val Leu Arg His Pro
            100                 105                 110

Thr Trp Pro Gln Arg Ser Val Trp His Gly Ser Asp Pro Asn Gly Arg
            115                 120                 125

Arg Leu Thr Glu Ser Tyr Cys Glu Thr Trp Arg Thr Glu Ala Pro Ser
    130                 135                 140

Ala Thr Gly Gln Ala Ser Ser Leu Leu Gly Gly Arg Leu Leu Gly Gln
145                 150                 155                 160

Ser Ala Ala Ser Cys His His Ala Tyr Ile Val Leu Cys Ile Glu Asn
                165                 170                 175

Ser Phe Met Thr Ala Ser Arg
                180

<210>   6
<211>   183
<212>   PRT
<213>   ÈË¹¤ÐòÁÐ

<220>
<223>   µ°°×ÖÊ±àÌå

<400>   6

Met His Ser His Gln Asp Phe Gln Pro Val Leu His Leu Val Ala Leu
1               5               10                  15

```
Asn Ser Pro Leu Ser Gly Gly Met Arg Gly Ile Arg Gly Ala Asp Phe
            20              25              30

Gln Cys Phe Gln Gln Ala Arg Ala Val Gly Leu Ala Gly Thr Phe Arg
            35              40              45

Ala Phe Leu Ser Ser Arg Leu Gln Asp Leu Tyr Ser Ile Val Arg Arg
    50              55              60

Ala Asp Arg Ala Ala Val Pro Ile Val Asn Leu Lys Asp Glu Leu Leu
65              70              75              80

Phe Pro Ser Trp Glu Ala Leu Phe Ser Ser Glu Gly Pro Leu Gln Pro
            85              90              95

Gly Ala Arg Ile Phe Ser Phe Asp Gly Lys Asp Val Leu Arg His Pro
            100             105             110

Thr Trp Pro Gln Lys Ser Val Trp His Gly Ser Asp Pro Asn Gly Arg
            115             120             125

Arg Leu Thr Glu Ser Tyr Cys Glu Thr Trp Arg Thr Glu Ala Pro Ser
    130             135             140

Ala Thr Gly Gln Ala Ser Ser Leu Leu Gly Gly Arg Leu Leu Gly Gln
145             150             155             160

Ser Ala Ala Ser Cys His His Ala Tyr Ile Val Leu Cys Ile Glu Asn
            165             170             175

Ser Phe Met Thr Ala Ser Lys
            180
```

```
<210>  7
<211>  181
<212>  PRT
<213>  ÈË¹¤ÐòÁÐ

<220>
<223>  µ°°×Ê±äÌå

<400>  7
```

```
Met Asp Phe Gln Pro Val Leu His Leu Val Ala Leu Asn Ser Pro Leu
1               5               10              15

Ser Gly Gly Met Arg Gly Ile Arg Gly Ala Asp Phe Gln Cys Phe Gln
            20              25              30
```

```
Gln Ala Arg Ala Val Gly Leu Ala Gly Thr Phe Arg Ala Phe Leu Ser
        35                  40                  45


Ser Arg Leu Gln Asp Leu Tyr Ser Ile Val Arg Arg Ala Asp Arg Ala
        50                  55                  60


Ala Val Pro Ile Val Asn Leu Lys Asp Glu Leu Leu Phe Pro Ser Trp
65                  70                  75                  80


Glu Ala Leu Phe Ser Gly Glu Ser Gly Ala Gly Lys Thr Pro Gly Ala
                85                  90                  95


Arg Ile Phe Ser Phe Asp Gly Lys Asp Val Leu Arg His Pro Thr Trp
            100                 105                 110


Pro Gln Lys Ser Val Trp His Gly Ser Asp Pro Asn Gly Arg Arg Leu
        115                 120                 125


Thr Glu Ser Tyr Cys Glu Thr Trp Arg Thr Glu Ala Pro Ser Ala Thr
    130                 135                 140


Gly Gln Ala Ser Ser Leu Leu Gly Gly Arg Leu Leu Gly Gln Ser Ala
145                 150                 155                 160


Ala Ser Cys His His Ala Tyr Ile Val Leu Cys Ile Glu Asn Ser Phe
                165                 170                 175


Met Thr Ala Ser Lys
                180


<210>   8
<211>   180
<212>   PRT
<213>   ÈË¹¤ÐòÁÐ

<220>
<223>   µ°°×ÖÊ±àÌå

<400>   8

Met Arg Asp Phe Gln Pro Val Leu His Leu Val Ala Leu Asn Ser Pro
1               5                   10                  15


Leu Ser Gly Gly Met Arg Gly Ile Arg Gly Ala Asp Phe Gln Cys Phe
            20                  25                  30


Gln Gln Ala Arg Gln Val Gly Leu Ala Gly Thr Phe Arg Ala Phe Leu
        35                  40                  45
```

```
Ser Ser Arg Leu Gln Asp Leu Tyr Ser Ile Val Arg Arg Ala Asp Arg
    50                  55                  60

Ala Ala Val Pro Ile Val Asn Leu Lys Asp Glu Leu Leu Phe Pro Ser
65                  70                  75                  80

Trp Glu Ala Leu Phe Ser Ser Glu Gly Pro Leu Lys Pro Gly Ala Arg
                85                  90                  95

Ile Phe Ser Phe Asp Gly Lys Asp Val Leu Arg His Pro Thr Trp Pro
                100                 105                 110

Gln Lys Ser Val Trp His Gly Ser Asp Pro Asn Gly Arg Arg Leu Thr
        115                 120                 125

Glu Ser Tyr Cys Glu Thr Trp Arg Thr Glu Ala Pro Ser Ala Thr Gly
    130                 135                 140

Gln Ala Ser Ser Leu Leu Gly Gly Arg Leu Leu Gly Gln Ser Ala Ala
145                 150                 155                 160

Ser Cys His His Ala Tyr Ile Val Leu Cys Ile Glu Asn Ser Phe Met
            165                 170                 175

Thr Ala Ser Lys
            180


<210>  9
<211>  181
<212>  PRT
<213>  ÈË¹¤ÐòÁÐ

<220>
<223>  µ°°×Öê±äÌå

<400>  9

Met Arg Asp Phe Gln Pro Val Leu His Leu Val Ala Leu Asn Ser Pro
1               5                   10                  15

Leu Ser Gly Gly Met Arg Gly Ile Arg Gly Ala Asp Phe Gln Cys Phe
        20                  25                  30

Gln Gln Ala Arg Ala Val Gly Leu Ala Gly Thr Phe Arg Ala Phe Leu
        35                  40                  45

Ser Ser Arg Leu Gln Asp Leu Tyr Ser Ile Val Arg Arg Ala Asp Arg
    50                  55                  60

Gly Ser Val Pro Ile Val Asn Leu Lys Asp Glu Val Leu Ser Pro Ser
```

|     |     | 65  |     |     |     | 70  |     |     |     | 75  |     |     |     | 80  |     |
|-----|-----|-----|-----|-----|-----|-----|-----|-----|-----|-----|-----|-----|-----|-----|-----|

Trp Asp Ser Leu Phe Ser Gly Ser Gln Gly Gln Leu Gln Pro Gly Ala
                    85                  90                  95

Arg Ile Phe Ser Phe Asp Gly Arg Asp Ile Leu Gln Asp Ser Ala Trp
            100                 105                 110

Pro Gln Lys Ser Val Trp His Gly Ser Asp Ala Lys Gly Arg Arg Leu
            115                 120                 125

Pro Glu Ser Tyr Cys Glu Ala Trp Arg Thr Asp Glu Arg Gly Thr Ser
            130                 135                 140

Gly Gln Ala Ser Ser Leu Leu Ser Gly Arg Leu Leu Glu Gln Lys Ala
145                 150                 155                 160

Ala Ser Cys His Asn Ser Tyr Ile Val Leu Cys Ile Glu Asn Ser Phe
                165                 170                 175

Met Thr Ala Ser Lys
                180

<210> 10
<211> 185
<212> PRT
<213> ÈË¹¤ÐòÁÐ

<220>
<223> µ°°×ÖÊ±äÌå

<400> 10

Met His Val His Gln Asp Phe Gln Pro Ala Leu His Leu Val Ala Leu
1                   5                   10                  15

Asn Thr Pro Leu Ser Gly Gly Met Arg Gly Ile Arg Gly Ala Asp Phe
            20                  25                  30

Gln Cys Phe Gln Gln Ala Arg Gln Val Gly Leu Ala Gly Thr Phe Arg
            35                  40                  45

Ala Phe Leu Ser Ser Arg Leu Gln Asp Leu Tyr Ser Ile Val Arg Arg
        50                  55                  60

Ala Asp Arg Thr Ala Val Pro Ile Val Asn Leu Arg Asp Glu Val Leu
65                  70                  75                  80

Phe Ser Asn Trp Glu Ala Leu Phe Thr Gly Ser Glu Ala Pro Leu Arg
                85                  90                  95

Ala Gly Ala Arg Ile Phe Ser Phe Asp Gly Arg Asp Val Leu Arg His
            100                     105                 110

Pro Thr Trp Pro Gln Lys Ser Val Trp His Gly Ser Asp Pro Asn Gly
            115                     120                 125

Arg Arg Leu Thr Glu Ser Tyr Cys Glu Thr Trp Arg Thr Glu Ala Pro
    130                     135                 140

Ser Ala Thr Gly Gln Ala Ser Ser Leu Leu Ala Gly Arg Leu Leu Glu
145                     150                 155                 160

Gln Lys Ala Ala Gly Cys His Asn Ala Phe Ile Val Leu Cys Ile Glu
                165                 170                 175

Asn Ser Phe Met Thr Ser Ser Ser Lys
            180                 185

<210> 11
<211> 186
<212> PRT
<213> ÈË¹¤ÐòÁÐ

<220>
<223> µ°°×ÖÊ±äÌå

<400> 11

Met His Thr His Gln Asp Phe His Pro Val Leu His Leu Val Ala Leu
1               5               10                  15

Asn Thr Pro Leu Ser Gly Gly Met Arg Gly Ile Arg Gly Ala Asp Phe
            20                  25                  30

Gln Cys Phe Gln Gln Ala Arg Ala Val Gly Leu Ser Gly Thr Phe Arg
            35                  40                  45

Ala Phe Leu Ser Ser Arg Leu Gln Asp Leu Tyr Ser Ile Val Arg Arg
    50                      55                  60

Ala Asp Arg Ala Ala Val Pro Ile Val Asn Leu Lys Asp Glu Leu Leu
65                      70                  75                  80

Phe Pro Ser Trp Glu Ala Leu Phe Ser Gly Glu Ser Gly Ala Gly Lys
                85                  90                  95

Thr Gly Gly Ala Arg Ile Phe Ser Phe Asp Gly Arg Asp Val Leu Arg
            100                     105                 110

His Pro Ala Trp Pro Gln Lys Ser Val Trp His Gly Ser Asp Pro Ser
115                 120                 125

Gly Arg Arg Leu Thr Glu Ser Tyr Cys Glu Thr Trp Arg Thr Asp Ser
130                 135                 140

Arg Ala Ala Thr Gly Gln Ala Ser Ser Leu Leu Ala Gly Arg Leu Leu
145                 150                 155                 160

Glu Gln Lys Ala Ala Gly Cys His Asn Ala Phe Ile Val Leu Cys Ile
                165                 170                 175

Glu Asn Ser Phe Met Thr Ser Ser Ser Lys
                180                 185


<210>  12
<211>  184
<212>  PRT
<213>  ÈË¹¤ÐòÁÐ

<220>
<223>  µ°°×ÖÊ±äÌå

<400>  12

Met His Ser His Arg Asp Phe Gln Pro Val Leu His Leu Val Ala Leu
1                   5                   10                  15

Asn Ser Pro Leu Ser Gly Gly Met Arg Gly Ile Arg Gly Ala Asp Phe
            20                  25                  30

Gln Cys Phe Gln Gln Ala Arg Ala Val Gly Leu Ala Gly Thr Phe Arg
            35                  40                  45

Ala Phe Leu Ser Ser Arg Leu Gln Asp Leu Tyr Ser Ile Val Arg Arg
            50                  55                  60

Ala Asp Arg Ala Ala Val Pro Ile Val Asn Leu Lys Asp Glu Leu Leu
65                  70                  75                  80

Phe Pro Ser Trp Glu Ala Leu Phe Ser Gly Ser Glu Gly Pro Leu Lys
                85                  90                  95

Pro Gly Ala Arg Ile Phe Ser Phe Asp Gly Arg Asp Val Leu Arg His
            100                 105                 110

Pro Thr Trp Pro Gln Lys Ser Val Trp His Gly Ser Asp Pro Ser Gly
            115                 120                 125

```
      His Arg Leu Thr Glu Ser Tyr Cys Glu Thr Trp Arg Thr Asp Ser Arg
          130                 135                 140

      Ala Ala Thr Gly Gln Ala Ser Ser Leu Leu Gly Gly Arg Leu Leu Gly
          145                 150                 155                 160

      Gln Ser Ala Ala Ser Cys His His Ala Tyr Ile Val Leu Cys Ile Ala
                      165                 170                 175

      Asn Ser Phe Met Thr Ala Ser Lys
                      180
```

<210> 13
<211> 180
<212> PRT
<213> ÈË¹¤ÐòÁÐ

<220>
<223> µ°°×ÖÊ±äÌå

<400> 13

```
      Met Asp Phe Gln Pro Val Leu His Leu Val Ala Leu Asn Ser Pro Leu
      1               5                   10                  15

      Ser Gly Gly Met Arg Gly Ile Arg Gly Ala Asp Phe Gln Cys Phe Gln
                      20                  25                  30

      Gln Ala Arg Ala Val Gly Leu Ala Gly Thr Phe Arg Ala Phe Leu Ser
                35                  40                  45

      Ser Arg Leu Gln Asp Leu Tyr Ser Ile Val Arg Arg Ala Asp Arg Ala
                50                  55                  60

      Ala Val Pro Ile Val Asn Leu Lys Asp Glu Leu Leu Phe Pro Ser Trp
      65                  70                  75                  80

      Glu Ala Leu Phe Ser Gly Ser Glu Gly Pro Leu Arg Pro Gly Ala Arg
                      85                  90                  95

      Ile Phe Ser Phe Asp Gly Lys Asp Val Leu Arg His Pro Thr Leu Pro
                      100                 105                 110

      Gln Lys Ser Val Trp His Gly Ser Asp Pro Ser Gly Arg Arg Leu Thr
                115                 120                 125

      Glu Ser Tyr Cys Glu Thr Trp Arg Thr Asp Ser Arg Ala Ala Thr Gly
          130                 135                 140

      Gln Ala Ser Ser Leu Leu Gly Gly Arg Leu Leu Gly Gln Ser Ala Ala
```

```
                145                    150                    155                    160


       Ser Cys His His Ala Tyr Ile Val Leu Cys Ile Glu Asn Ser Phe Met
                       165                    170                    175


       Thr Ala Ser Lys
                   180


<210>  14
<211>  181
<212>  PRT
<213>  ĚË¹¤ÐòÁÐ

<220>
<223>  µ°°×ÖÊ±äÌå

<400>  14

       Met Asp Phe Gln Pro Val Leu His Leu Val Ala Leu Asn Ser Pro Leu
       1               5                   10                     15


       Ser Gly Gly Met Arg Gly Ile Arg Gly Ala Asp Phe Gln Cys Phe Gln
                   20                  25                     30


       Gln Ala Arg Ala Val Gly Leu Ala Gly Thr Phe Arg Ala Phe Leu Ser
                   35                  40                     45


       Ser Arg Leu Gln Asp Leu Tyr Ser Ile Val Arg Arg Ala Asp Arg Ala
                   50                  55                     60


       Ala Val Pro Ile Val Asn Leu Lys Asp Glu Leu Leu Phe Pro Ser Trp
       65                  70                     75                     80


       Glu Ala Leu Phe Ser Gly Ser Glu Gly Pro Leu Arg Pro Gly Ala Arg
                       85                  90                     95


       Ile Phe Ser Phe Asp Gly Lys Asp Val Leu Arg His Pro Thr Leu Pro
                   100                     105                    110


       Gln Lys Ser Val Trp His Gly Ser Asp Pro Ser Gly Arg Arg Leu Thr
                   115                     120                    125


       Glu Ser Tyr Cys Glu Thr Trp Arg Thr Asp Ser Arg Ala Ala Thr Gly
                   130                     135                    140


       Gln Ala Ser Ser Leu Leu Gly Gly Arg Leu Leu Gly Gln Ser Ala Ala
       145                     150                    155                    160


       Ser Cys His His Ala Tyr Ile Val Leu Cys Ile Glu Asn Ser Phe Met
                       165                    170                    175
```

```
Thr Ala Ser Lys Lys
            180
```

```
<210>  15
<211>  185
<212>  PRT
<213>  ÈË¹¤ÐòÁÐ

<220>
<223>  µ°°×ÖÊ±äÌå

<400>  15
```

```
Met His Thr His Gln Asp Phe His Pro Val Leu His Leu Val Ala Leu
1                   5                   10                  15
```

```
Asn Thr Pro Leu Ser Gly Gly Met Arg Gly Ile Arg Gly Ala Asp Phe
            20                  25                  30
```

```
Gln Cys Phe Gln Gln Ala Arg Ala Val Gly Leu Ala Gly Thr Phe Arg
            35                  40                  45
```

```
Ala Phe Leu Ser Ser Arg Leu Gln Asp Leu Tyr Ser Ile Val Arg Arg
            50                  55                  60
```

```
Ala Asp Arg Ala Ala Val Pro Ile Val Asn Leu Lys Asp Glu Leu Leu
65                  70                  75                  80
```

```
Phe Pro Ser Trp Glu Ala Leu Phe Ser Gly Ser Glu Gly Pro Leu Lys
                85                  90                  95
```

```
Pro Gly Ala Arg Ile Phe Ser Phe Asp Gly Arg Asp Ile Leu Gln Asp
            100                 105                 110
```

```
Ser Ala Trp Pro Gln Lys Ser Val Trp His Gly Ser Asp Pro Asn Gly
            115                 120                 125
```

```
Arg Arg Leu Thr Glu Ser Tyr Cys Glu Thr Trp Arg Thr Glu Ala Pro
            130                 135                 140
```

```
Ser Ala Thr Gly Gln Ala Ser Ser Leu Ser Ser Gly Lys Leu Leu Glu
145                 150                 155                 160
```

```
Gln Ser Val Ser Ser Cys Gln His Ala Phe Val Val Leu Cys Ile Glu
                165                 170                 175
```

```
Asn Ser Phe Met Thr Ala Ala Lys Lys
                180                 185
```

```
<210>   16
<211>   183
<212>   PRT
<213>   ÈË¹¤ÐòÁÐ

<220>
<223>   µ°°×Ðʱäìå

<400>   16
```

Met His Thr His Thr Ser Gly Pro Gly Leu His Leu Ile Ala Leu Asn
1               5                   10                  15

Ser Pro Gln Val Gly Asn Met Arg Gly Ile Arg Gly Ala Asp Phe Gln
            20                  25                  30

Cys Phe Gln Gln Ala Arg Ala Val Gly Leu Ala Gly Thr Phe Arg Ala
        35                  40                  45

Phe Leu Ser Ser Arg Leu Gln Asp Leu Tyr Ser Ile Val Arg Arg Ala
        50                  55                  60

Asp Arg Ser Ser Val Pro Ile Val Asn Leu Lys Asp Glu Val Leu Ser
65                  70                  75                  80

Pro Ser Trp Asp Ser Leu Phe Ser Val Ser Gln Gly Gln Leu Gln Pro
                85                  90                  95

Gly Ala Arg Ile Phe Ser Phe Asp Gly Arg Asp Ile Leu Gln Asp Ser
                100                 105                 110

Ala Trp Pro Gln Lys Ser Val Trp His Gly Ser Asp Pro Asn Gly Arg
        115                 120                 125

Arg Leu Thr Glu Ser Tyr Cys Glu Thr Trp Arg Thr Glu Ala Pro Ser
        130                 135                 140

Ala Thr Gly Gln Ala Ser Ser Leu Leu Gly Gly Arg Leu Leu Gly Gln
145                 150                 155                 160

Ser Ala Ala Ser Cys His His Ala Tyr Ile Val Leu Cys Ile Glu Asn
                165                 170                 175

Ser Phe Met Thr Ala Ser Lys
                180

```
<210>   17
<211>   184
<212>   PRT
<213>   ÈË¹¤ÐòÁÐ
```

EP 3 246 043 A1

<220>
<223>  µ°°×ÖÊ±äÌå

<400>  17

Met Thr Pro Thr Trp Tyr Pro Arg Met Leu Arg Val Ala Ala Leu Asn
1               5               10              15

Glu Pro Ser Thr Gly Asp Leu Gln Gly Ile Arg Gly Ala Asp Phe Gln
            20              25              30

Cys Phe Gln Gln Ala Arg Ala Val Gly Leu Ser Gly Thr Phe Arg Ala
        35              40              45

Phe Leu Ser Ser Arg Leu Gln Asp Leu Tyr Ser Ile Val Arg Arg Ala
    50              55              60

Asp Arg Ala Ala Val Pro Ile Val Asn Leu Lys Asp Glu Val Leu Ser
65              70              75              80

Pro Ser Trp Asp Ser Leu Phe Ser Gly Ser Gln Gly Gln Leu Gln Pro
            85              90              95

Gly Ala Arg Ile Phe Ser Phe Asp Gly Lys Asp Val Leu Arg His Pro
            100             105             110

Thr Trp Pro Gln Lys Ser Val Trp His Gly Ser Asp Pro Ser Gly Arg
        115             120             125

Arg Leu Met Glu Ser Tyr Cys Glu Thr Trp Arg Thr Glu Thr Thr Gly
    130             135             140

Ala Thr Gly Gln Ala Ser Ser Leu Leu Gly Gly Arg Leu Leu Gly Gln
145             150             155             160

Ser Ala Ala Ser Cys His His Ala Tyr Ile Val Leu Cys Ile Glu Asn
            165             170             175

Ser Phe Met Thr Asn Asn Arg Lys
            180

**Claims**

1. Use of endostatin or a functional variant thereof in the preparation of a medicament for treating dietary obesity, non-alcoholic fatty liver disease, insulin resistance or glucose intolerance.

2. The use of claim 1, wherein the functional variant is selected from the group consisting of: YH-16, 003, 007, Z101, ES006, ES008, ES011, S02, S09, Z006, Z008, ZN1, 009, S03, 36, 249, 381,57, 114, 124, 125, 160, 163, 119, mES, mYH-16, m003, m007, mZ101, mES006, mES008, mES011, mS02, mS09, mZ006, mZ008, mZN1, m009, mS03, m36, m249, m381, m57, m114, m124, m125, m160, m163, and m119.

3. The use of claim 1, wherein the functional variant is selected from the group consisting of: YH-16, 003, 007, Z101, 009, S03, 36, 249, mES, mYH-16, m003, m007, mZ101, m009, mS03, m36, and m249.

4. Use of endostatin or a functional variant thereof in the preparation of a medicament for inhibiting adipocyte differentiation.

5. The use of claim 4, wherein the functional variant is selected from the group consisting of: YH-16, 003, 007, Z101, ES006, ES008, ES011, S02, S09, Z006, Z008, ZN1, 009, S03, 36, 249, 381,57, 114, 124, 125, 160, 163, 119, mES, mYH-16, m003, m007, mZ101, mES006, mES008, mES011, mS02, mS09, mZ006, mZ008, mZN1, m009, mS03, m36, m249, m381, m57, m114, m124, m125, m160, m163, and m119.

6. The use of claim 4, wherein the functional variant is selected from the group consisting of: YH-16, 003, 007, Z101, 009, S03, 36, 249, mES, mYH-16, m003, m007, mZ101, m009, mS03, m36, and m249.

7. A method for treating dietary obesity, non-alcoholic fatty liver disease, insulin resistance or glucose intolerance, comprising administering to a subject a therapeutically effective amount of endostatin or a functional variant thereof.

8. The method of claim 7, wherein the functional variant is selected from the group consisting of: YH-16, 003, 007, Z101, ES006, ES008, ES011, S02, S09, Z006, Z008, ZN1, 009, S03, 36, 249, 381, 57, 114, 124, 125, 160, 163, 119, mES, mYH-16, m003, m007, mZ101, mES006, mES008, mES011, mS02, mS09, mZ006, mZ008, mZN1, m009, mS03, m36, m249, m381, m57, m114, m124, m125, m160, m163, and m119.

9. The method of claim 7, wherein the functional variant is selected from the group consisting of: YH-16, 003, 007, Z101, 009, S03, 36, 249, mES, mYH-16, m003, m007, mZ101, m009, mS03, m36, and m249.

10. A method for inhibiting adipocyte differentiation, comprising administering to a subject a therapeutically effective amount of endostatin or a functional variant thereof.

11. The method of claim 10, wherein the functional variant is selected from the group consisting of: YH-16, 003, 007, Z101, ES006, ES008, ES011, S02, S09, Z006, Z008, ZN1, 009, S03, 36, 249, 381, 57, 114, 124, 125, 160, 163, 119, mES, mYH-16, m003, m007, mZ101, mES006, mES008, mES011, mS02, mS09, mZ006, mZ008, mZN1, m009, mS03, m36, m249, m381, m57, m114, m124, m125, m160, m163, and m119.

12. The method of claim 10, wherein the functional variant is selected from the group consisting of: YH-16, 003, 007, Z101, 009, S03, 36, 249, mES, mYH-16, m003, m007, mZ101, m009, mS03, m36, and m249.

13. A medicament for treating dietary obesity, non-alcoholic fatty liver disease, insulin resistance or glucose intolerance, comprising endostatin or a functional variant thereof as active ingredient.

14. The medicament of claim 13, wherein the functional variant is selected from the group consisting of: YH-16, 003, 007, Z101, ES006, ES008, ES011, S02, S09, Z006, Z008, ZN1, 009, S03, 36, 249, 381, 57, 114, 124, 125, 160, 163, 119, mES, mYH-16, m003, m007, mZ101, mES006, mES008, mES011, mS02, mS09, mZ006, mZ008, mZN1, m009, mS03, m36, m249, m381, m57, m114, m124, m125, m160, m163, and m119.

15. The medicament of claim 13, wherein the functional variant is selected from the group consisting of: YH-16, 003, 007, Z101, 009, S03, 36, 249, mES, mYH-16, m003, m007, mZ101, m009, mS03, m36, and m249.

Figure 1A

Figure 1B

33

Figure 1C

Figure 2A

Normal diet      High fat diet      High fat diet + ES    High fat diet + YH-16

Figure 2B

Figure 2C

Figure 3A

Figure 3B

Figure 3C

Figure 4A

Figure 4 B

Figure 4C

Figure 4D

Figure 5A

Figure 5B

Figure 5C

Figure 6A

Figure 6B

Figure 6C

Figure 7A

Figure 7B

Figure 8A

Figure 8B

Figure 8C

Figure 8D

Figure 9A

Figure 9B

Figure 10A

Figure 10B

Figure 10C

Figure 10D

Figure 11A

Figure 11 B

Figure 12A

Figure 12B

Figure 12C

Figure 12D

(M) H S H R D F Q P V L H L V A L N S P L S G G M R G I R G A D F Q C F Q Q A R
A V G L A G T F R A F L S S R L Q D L Y S I V R R A D R A A V P I V N L K D E L L
F P S W E A L F S A S E G P L K P G A R I F S F D G K D V L R H P T W P Q K S V
W H G S D P N G R R L T E S Y C E T W R T E A P S A T G Q A S S L L G G R L L
G Q S A A S C H H A Y I V L C I E N S F M T A S K

Figure 13

(M)HSHRDFQPVLHLVALNSPLSGGMRGIRGADFQCFQQARAVGLAGTFRAFLSSRLQDLYSI
VRRADRAAVPIVNLKDELLFPSWEALFSASKAPLRPGARIFSFDGKDVLRHPTWPQKSVWHGS
DPNGRRLTESYCETWRTEAPSATGQASSLLGGRLLGQSAASCHHAYIVLCIENSFMTASK

Figure 14

MHSHRDFQPVLHLVALNSPLSGGMRG
IRGADFQCFQQARAVGLAGTFRAFLSS
RLQDLYSIVRRADRAAVPIVNLKDELLF
PSWEALFSSEGPLKPGARIFSFDGRDVL
RHPTWPQRSVWHGSDPNGRRLTESYC
ETWRTEAPSATGQASSLLGGRLLGQSA
ASCHHAYIVLCIENSFMTASR

Figure 15

(M)HSHQDFQPVLHLVALNSPLSGGMRGIRGADFQCFQQARAVGLAGTFRAFLSSRLQDLYSI
VRRADRAAVPIVNLKDELLFPSWEALFSSEGPLQPGARIFSFDGKDVLRHPTWPQKSVWHGS
DPNGRRLTESYCETWRTEAPSATGQASSLLGGRLLGQSAASCHHAYIVLCIENSFMTASK

Figure 16

(M)DFQPVLHLVALNSPLSGGMRGIRGADFQCFQQARAVGLAGTFRAFLSSRLQDLYSIVRRA
DRAAVPIVNLKDELLFPSWEALFSGESGAGKTPGARIFSFDGKDVLRHPTWPQKSVWHGSDP
NGRRLTESYCETWRTEAPSATGQASSLLGGRLLGQSAASCHHAYIVLCIENSFMTASK

Figure 17

(M)RDFQPVLHLVALNSPLSGGMRGIRGADFQCFQQARQVGLAGTFRAFLSSRLQDLYSIVRR
ADRAAVPIVNLKDELLFPSWEALFSSEGPLKPGARIFSFDGKDVLRHPTWPQKSVWHGSDPNG
RRLTESYCETWRTEAPSATGQASSLLGGRLLGQSAASCHHAYIVLCIENSFMTASK

Figure 18

(M)RDFQPVLHLVALNSPLSGGMRGIRGADFQCFQQARAVGLAGTFRAFLSSRLQDLYSIVRR
ADRGSVPIVNLKDEVLSPSWDSLFSGSQGQLQPGARIFSFDGRDILQDSAWPQKSVWHGSDA
KGRRLPESYCEAWRTDERGTSGQASSLLSGRLLEQKAASCHNSYIVLCIENSFMTASK

Figure 19

(M)HSHRDFQPVLHLVALNSPLSGGMRGIRGADFQCFQQARAVGLAGTFRAFLSSRLQDLYSIVRR
ADRAAVPIVNLKDELLFPSWEALFSGSEGPLKPGARIFSFDGKDVLRHPTWPQKSVWHGSDPNGR
RLTESYCETWRTEAPSATGQASSLLGGRLLGQSAASCHHAYIVLCIENSFMTASK

Figure 20

(M)GGSHHHHHHSHRDFQPVLHLVALNSPLSGGMRGIRGADFQCFQQARAVGLAGTFRAFLS
SRLQDLYSIVRRADRAAVPIVNLKDELLFPSWEALFSGSEGPLKPGARIFSFDGKDVLRHPTWP
QKSVWHGSDPNGRRLTESYCETWRTEAPSATGQASSLLGGRLLGQSAASCHHAYIVLCIENSF
MTASK

Figure 21

381

(M)HVHQDFQPALHLVALNTPLSGGMRGIRGADFQCFQQARQVGLAGTFRAFLSSRLQDLYSI
VRRADRTAVPIVNLRDEVLFSNWEALFTGSEAPLRAGARIFSFDGRDVLRHPTWPQKSVWHG
SDPNGRRLTESYCETWRTEAPSATGQASSLLAGRLLEQKAAGCHNAFIVLCIENSFMTSSSK

57

(M)HTHQDFHPVLHLVALNTPLSGGMRGIRGADFQCFQQARAVGLSGTFRAFLSSRLQDLYSI
VRRADRAAVPIVNLKDELLFPSWEALFSGESGAGKTGGARIFSFDGRDVLRHPAWPQKSVWH
GSDPSGRRLTESYCETWRTDSRAATGQASSLLAGRLLEQKAAGCHNAFIVLCIENSFMTSSSK

114

(M)HSHRDFQPVLHLVALNSPLSGGMRGIRGADFQCFQQARAVGLAGTFRAFLSSRLQDLYSI
VRRADRAAVPIVNLKDELLFPSWEALFSGSEGPLKPGARIFSFDGRDVLRHPTWPQKSVWHGS
DPSGHRLTESYCETWRTDSRAATGQASSLLGGRLLGQSAASCHHAYIVLCIANSFMTASK

124

(M)DFQPVLHLVALNSPLSGGMRGIRGADFQCFQQARAVGLAGTFRAFLSSRLQDLYSIVRRA
DRAAVPIVNLKDELLFPSWEALFSGSEGPLRPGARIFSFDGKDVLRHPTLPQKSVWHGSDPSG
RRLTESYCETWRTDSRAATGQASSLLGGRLLGQSAASCHHAYIVLCIENSFMTASK

Figure 22

125

(M)DFQPVLHLVALNSPLSGGMRGIRGADFQCFQQARAVGLAGTFRAFLSSRLQDLYSIVRRA
DRAAVPIVNLKDELLFPSWEALFSGSEGPLRPGARIFSFDGKDVLRHPTLPQKSVWHGSDPSG
RRLTESYCETWRTDSRAATGQASSLLGGRLLGQSAASCHHAYIVLCIENSFMTASKK

160

(M)HTHQDFHPVLHLVALNTPLSGGMRGIRGADFQCFQQARAVGLAGTFRAFLSSRLQDLYSI
VRRADRAAVPIVNLKDELLFPSWEALFSGSEGPLKPGARIFSFDGRDILQDSAWPQKSVWHGS
DPNGRRLTESYCETWRTEAPSATGQASSLSSGKLLEQSVSSCQHAFVVLCIENSFMTAAKK

163

(M)TPTWYPRMLRVAALNEPSTGDLQGIRGADFQCFQQARAVGLSGTFRAFLSSRLQDLYSIV
RRADRAAVPIVNLKDEVLSPSWDSLFSGSQGQLQPGARIFSFDGKDVLRHPTWPQKSVWHGS
DPSGRRLMESYCETWRTETTGATGQASSLLGGRLLGQSAASCHHAYIVLCIENSFMTNNRK

119

(M)HTHTSGPGLHLIALNSPQVGNMRGIRGADFQCFQQARAVGLAGTFRAFLSSRLQDLYSIV
RRADRSSVPIVNLKDEVLSPSWDSLFSVSQGQLQPGARIFSFDGRDILQDSAWPQKSVWHGS
DPNGRRLTESYCETWRTEAPSATGQASSLLGGRLLGQSAASCHHAYIVLCIENSFMTASK

Figure 23

## INTERNATIONAL SEARCH REPORT

| International application No. |
| --- |
| **PCT/CN2015/093726** |

**A.   CLASSIFICATION OF SUBJECT MATTER**

A61K 38/17 (2006.01) i; A61P 1/16 (2006.01) i; A61P 3/04 (2006.01) i; A61P 5/50 (2006.01) i

According to International Patent Classification (IPC) or to both national classification and IPC

**B.   FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, CPRSABS, SIPOABS, DWPI, CNTXT, WOTXT, EPTXT, USTXT, CNKI, GOOGLE SCHOLAR, Elsevier Science, PubMed: Endostatin, ES, YH-16, Z101, obesity, non-alcoholic fatty liver disease, insulin resistance or glucose intolerance, adipocyte differentiation

GenBank, EMBL: sequence search on the basis of SEQ ID NOs: 1-17

**C.   DOCUMENTS CONSIDERED TO BE RELEVANT**

| Category* | Citation of document, with indication, where appropriate, of the relevant passages | Relevant to claim No. |
| --- | --- | --- |
| X | CN 103703140 A (THE BOARD OF REGENTS OF THE UNIVERSITY OF TEXAS SYSTEM), 02 April 2014 (02.04.2014), see description, paragraphs 9-18 and 31-32 | 1, 4, 13 |
| Y | CN 103703140 A (THE BOARD OF REGENTS OF THE UNIVERSITY OF TEXAS SYSTEM), 02 April 2014 (02.04.2014), see description, paragraphs 9-18 and 31-32 | 2, 3, 5, 6 |
| X | CN 101596320 A (TSINGHUA UNIVERSITY; PROTGEN LTD.), 09 December 2009 (09.12.2009), see claim 38 | 13-15 |
| Y | CN 101596320 A (TSINGHUA UNIVERSITY; PROTGEN LTD.), 09 December 2009 (09.12.2009), see claim 38 | 2, 3, 5, 6 |

☐ Further documents are listed in the continuation of Box C.     ☒   See patent family annex.

| *        Special categories of cited documents: | "T"    later document published after the international filing date or priority date and not in conflict with the application but cited to understand the principle or theory underlying the invention |
| --- | --- |
| "A"    document defining the general state of the art which is not considered to be of particular relevance | |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
| --- | --- |
| 01 February 2016 (01.02.2016) | **14 February 2016 (14.02.2016)** |

| Name and mailing address of the ISA/CN:<br>State Intellectual Property Office of the P. R. China<br>No. 6, Xitucheng Road, Jimenqiao<br>Haidian District, Beijing 100088, China<br>Facsimile No.: (86-10) 62019451 | Authorized officer<br><br>**ZHAO, Yanhao**<br><br>Telephone No.: (86-10) **62411043** |

Form PCT/ISA/210 (second sheet) (July 2009)

## INTERNATIONAL SEARCH REPORT

International application No.

**PCT/CN2015/093726**

**Box No. II    Observations where certain claims were found unsearchable (Continuation of item 2 of first sheet)**

This international search report has not been established in respect of certain claims under Article 17(2)(a) for the following reasons:

1. ☒   Claims Nos.: 7-12
because they relate to subject matter not required to be searched by this Authority, namely:
[1] claims 7-12 relate to a treatment method of the human or animal body. (PCT Rule 39.1(iv))。

2. ☐   Claims Nos.:
because they relate to parts of the international application that do not comply with the prescribed requirements to such an extent that no meaningful international search can be carried out, specifically:

3. ☐   Claims Nos.:
because they are dependent claims and are not drafted in accordance with the second and third sentences of Rule 6.4(a).

Form PCT/ISA/210 (continuation of first sheet (2)) (July 2009)

## INTERNATIONAL SEARCH REPORT
### Information on patent family members

International application No.

**PCT/CN2015/093726**

| Patent Documents referred in the Report | Publication Date | Patent Family | Publication Date |
|---|---|---|---|
| CN 103703140 A | 02 April 2014 | JP 2014510758 A | 01 May 2014 |
| | | CA 2830375 A1 | 04 October 2012 |
| | | AU 2012236199 A1 | 26 September 2013 |
| | | WO 2012135729 A9 | 30 January 2014 |
| | | US 2014243258 A1 | 28 August 2014 |
| | | EP 2704736 A2 | 12 March 2014 |
| | | MX 2013011294 A | 06 October 2014 |
| | | WO 2012135729 A2 | 04 October 2012 |
| | | EA 201391154 A2 | 30 June 2014 |
| | | IL 228617 D0 | 31 December 2013 |
| | | US 9029321 B2 | 12 May 2015 |
| | | SG 193317 A1 | 30 October 2013 |
| | | KR 20140026408 A | 05 March 2014 |
| CN 101596320 A | 09 December 2009 | CN 101596320 B | 09 January 2013 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2012081210 W

### Non-patent literature cited in the description

- **CRISTANCHO AG et al.** *Nat Rev Mol Cell Biol,* 2011, vol. 12, 722-734
- **DAQUINAG AC et al.** *Trends Pharmacol Sci,* 2011, vol. 32, 300-307
- **O'REILLY MS et al.** *Cell,* 1997, vol. 88, 277-285
- **BOEHM T. et al.** *Nature,* vol. 197 (390), 404-407
- **FU Y et al.** *IUBMB Life,* 2009, vol. 61, 613-626
- **WANG J et al.** *Zhongguo fei ai za zhi,* 2005, vol. 8, 283-290
- **HAN B et al.** *J Thorac Oncol,* 2011, vol. 6 (6), 1104-1109
- **RUPNICK MA et al.** *Proc Natl Acad Sci U S A,* 2002, vol. 99, 10730-10735
- **KIM MY et al.** *Int J Obes (Lond).,* 2010, vol. 34, 820-830
- **TANG QQ et al.** *Annu Rev Biochem,* 2012, vol. 81, 715-736
- **LEE J et al.** *J Cell Biochem,* 2012, vol. 113, 2488-2499
- **MALIK VS et al.** *Nat Rev Endocrinol,* 2013, vol. 9, 13-27